# EUROPEAN PATENT APPLICATION

(11) **EP 4 458 370 A1**
(43) Date of publication of application: **06.11.2024**
(21) Application number: 22916815.8
(22) Date of filing: 29.12.2022
(51) Int. Cl.: A61K 38/17, A61P 27/02, A61P 27/04

(54) **COMPOSITION FOR PREVENTION OR TREATMENT OF DRY EYE DISEASE COMPRISING HAPLN1**

(30) Priority: 30.12.2021 KR 20210193440
(71) Applicant: HAPLNSCIENCE INC., Gyeonggi-do 13494 (KR)
(72) Inventor: KIM, Dae Kyong, Seongnam-si, Gyeonggi-do 13547 (KR); KIM, Yong Soon, Seoul 04778 (KR); BACK, Moon Jung, Seongnam-si, Gyeonggi-do 13489 (KR); CHAE, Dong Kyu, Yongin-si, Gyeonggi-do 16918 (KR); PYO, Jung Hoon, Seongnam-si, Gyeonggi-do 13504 (KR); KIM, David, Suwon-si, Gyeonggi-do 16842 (KR)
(74) Representative: Mewburn Ellis LLP
(86) International application number: PCT/KR2022/021657
(87) International publication number: WO 2023/128674

(57) **Abstract**

Disclosed are a pharmaceutical composition for prevention or treatment of dry eye disease, including hyaluronan and proteoglycan link protein 1 (HAPLN1) as an active ingredient, and a method for preventing or treating dry eye disease by using same. According to the present disclosure, dry eye disease may be effectively and stably prevented or treated, and side effects are reduced as compared to conventional products.

## Description

### Technical Field

The present disclosure relates to the field of a medical approach for treating dry eye disease. The present disclosure also relates to a pharmaceutical composition for prevention and treatment of dry eye disease and a method of treating dry eye disease.

### Background Art

The eyeball is a complex organ including different specialized cells and tissues controlled to maintain optimal visual function. This function is achieved by a rigid barrier in the anterior and posterior parts of the eyeball which plays an important role in selective control of the passage of fluid and solutes into and/or out of the eyeball. Local and systemic disease may affect different regions of the eyeball, but it is difficult to develop effective drugs therefor due to anatomic characteristics of the eyeball and complex physiological characteristics of the retina and the optic nerve. The eyeball is broadly divided into two parts: an anterior segment (front side of the eyeball) and a posterior segment (back side of the eyeball). The anterior segment of the eyeball refers to the cornea, conjunctiva, anterior sclera (a part of sclera that transitions forward from edges to the cornea), iris, ciliary body, aqueous humour, and lens. The posterior segment of the eyeball (also called posterior eye segment) includes anterior vitreous membrane and a structure behind this membrane, posterior sclera (that transitions backward to optic nerve dura mater), vitreous body (including vitreous humor and membrane), retina, macula retina, choroid, and the optic nerve.

Dry eye disease (DED) is a disease accompanied by symptoms such as stinging eyes, scratching sensation, dry eyes, rough ocular surface, redness of eyes, abnormal secretion of tear due to blockage of tear duct, sensitivity to light, eye fatigue, discomfort when wearing contact lens, blurry vision, and foreign body sensation, and the number of patients has recently increased worldwide. For reference, according to US statistics, 29 million patients with dry eye disease are expected in 2022 and 6.6 billion dollars will be spent on treatment of dry eye disease in 2027. Even in Korea, the number of patients with dry eye disease is 2.4 million or more every year. Such an increasing trend of dry eye disease is considered to be affected by digitalization, frequent use of electronic devices such as smartphones in everyday life, and worsening surrounding environments.

Although new products are being developed to alleviate and treat dry eye disease, it is difficult to completely relieve or treat the symptoms. Direct causes of dry eye disease are lacrimal hyposecretion or hyperevaporation of tear. Although dry eye disease is not a life-threatening disease, patients experience severe discomfort with reduced visual function, and quality of life and productivity significantly deteriorate.

As a result of intensive research on HAPLN1 protein, the present inventors have found that external administration of a composition thereof may have efficacy on the prevention and treatment of dry eye disease, thereby completing the present disclosure.

In relation to HAPLN1, for example, Patent Document 1 discloses a configuration for differentiating cells into chondrocytes and chondroid cells by delivering thereto an expression vector encoding at least one therapeutic polynucleotide, and/or by producing additional cells by stimulating chondrocytes in joints via the delivery. In the detailed description thereof, HAPLN1 is disclosed among about 200 protein examples of the gene used. However, dry eye disease is not disclosed therein.

Furthermore, Patent Document 2, which discloses metallic nanoparticles coated with glycosaminoglycan and use thereof, relates to a composition for treating symptoms of dry eye disease. However, the composition includes a plurality of nanoparticles coated with a plurality of linker molecules, at least some of which are attached to at least one glycosaminoglycan chain, wherein HAPLN1 protein is a linker, the glycosaminoglycan chain includes hyaluronan and/or derivatives thereof, and the disclosed composition may be formulated for topical treatment. That is, Patent Document 2 discloses a configuration of introducing a hydrated glycosaminoglycan chain into skin or eyeballs by using HAPLN1 protein as a linker. However, because a nanoparticle/HAPLN1 conjugate is disclosed as the configuration of the disclosure, the HAPLN1 protein itself is not used as a main ingredient which is different from the function of the HAPLN1 protein acting independently to relieve dry eye disease by promoting secretion of tears by increasing expression of the mucin gene.

In addition, Non-patent Document 1 is a paper titled "Genetic Rescue of Chondrodysplasia and the Perinatal Lethal Effect of Cartilage Link Protein Deficiency" and discloses that targeted disruption of cartilage link protein gene (Crtl1) in mice resulted in chondrodysplasia and perinatal lethality, but overexpression of a cartilage-specific link protein introduced into Crtl1 null mice prevents the formation of abnormal chondrogenesis and reduces perinatal lethality, and Crtl1 transcripts and corresponding proteins were found in every organ tested from mouse embryos to adults. However, dry eye disease is not disclosed therein, either.

Meanwhile, examples of existing pharmaceutical compositions for relieving dry eye disease are Restasis^{®} (2003, Allergan), Cequa^{®} (2018, Sun), Xiidra^{®} (2016, Novartis), and Eysuvis^{®} (2020, Kala) by using immunosuppression or mechanisms inhibiting T cell migration. However, about 30 to 70 % of patients with dry eye disease stop taking drugs in the middle of treatment due to insufficient efficacy and side effects.

### [Related Art Document(s)]

### [Patent Document(s)]

Patent Document 1: US Patent Application Publication No. US2016/0220699
Patent Document 2: WO Patent Application Publication No. WO2016/156788

### [Non-patent Document(s)]

Non-patent Document 1: Journal of Biological Chemistry. 2003. Vol. 278, No 40. pp 39214-29223

### Disclosure

### Technical problem

As described above, conventional products for preventing and treating dry eye disease had insufficient efficacy and side effects. To overcome limitations of the related art, the present disclosure provides a pharmaceutical composition for effectively and stably treat dry eye disease and a method for treating dry eye disease.

### Technical Solution

According to an aspect of the present disclosure, a pharmaceutical composition for preventing or treating dry eye disease includes hyaluronan and proteoglycan link protein 1 (HAPLN1) or a gene encoding HAPLN1 as an active ingredient.

According to an embodiment of the present disclosure, the protein may have a sequence identity of 80 % or more to an amino acid sequence of SEQ ID NO: 1.

According to an embodiment of the present disclosure, a nucleic acid of the gene may be included in an expression vector.

According to an embodiment of the present disclosure, the pharmaceutical composition of the present disclosure may induce wound healing in cells in an eyeball.

According to an embodiment of the present disclosure, the pharmaceutical composition of the present disclosure may reduce expression of inflammatory factors of a corneal epithelial cell.

According to an embodiment of the present disclosure, the pharmaceutical composition of the present disclosure may increase an expression level of mucin.

According to an embodiment of the present disclosure, a single topical dose of the pharmaceutical composition of the present disclosure may be 1 ng/ml to 100 µg/ml.

According to an embodiment of the present disclosure, the pharmaceutical composition of the present disclosure may be in a formulation form selected from the group consisting of eye drops, artificial tears, ophthalmic ointments, tablets, pills, capsules, troches, inhalants, injections, patches, and suppositories.

According to an embodiment of the present disclosure, the pharmaceutical composition of the present disclosure may be for parenteral administration.

According to an embodiment of the present disclosure, the pharmaceutical composition of the present disclosure may further include a pharmaceutical additive selected from the group consisting of pharmaceutically acceptable carriers, diluents, binders, disintegrants, lubricants, and combinations thereof.

According to an embodiment of the present disclosure, the pharmaceutical composition of the present disclosure may be included as a major or minor active ingredient in a composition for preventing or suppressing dry eye disease.

According to another aspect of the present disclosure, a kit includes the composition of the present disclosure and administration instructions thereof.

According to another aspect of the present disclosure, a reagent composition for an experiment related to prevention or suppression of dry eye disease includes the composition of the present disclosure.

According to another aspect of the present disclosure, A use of hyaluronan and proteoglycan link protein 1 (HAPLN1) or a gene encoding HAPLN1 used to prepare a pharmaceutical composition for preventing or suppressing dry eye disease.

According to another aspect of the present disclosure, a method of preventing or treating dry eye disease includes administering hyaluronan and proteoglycan link protein 1 (HAPLN1) or a gene encoding HAPLN1, as an active ingredient, to an individual.

Furthermore, according to another aspect of the present disclosure, a use of a composition including hyaluronan and proteoglycan link protein 1 (HAPLN1) or a gene encoding the same as an active ingredient for preventing or treating dry eye disease.

### Advantageous Effects

As described above, according to the pharmaceutical composition and the treatment method therefor, dry eye disease may effectively and stably be prevented and treated with reduced side effects compared to existing products. This is because the HAPLN1 protein that is an active ingredient of the composition of the present disclosure is not a synthetic molecule but a biomolecule.

### Description of Drawings

FIG. 1A is a diagram illustrating an experiment plan according to Example 1 of the present disclosure.
FIGS. 1B and 1C are graphs showing results of an experiment conducted in accordance with the experiment plan of FIG. 1A of the present disclosure, indicating cell viability after 24 hours (FIG. 1B) and 48 hours (FIG. 1C) from administration of the composition according to the present disclosure, among the effects of the HAPLN1 protein on the relief of dry eye disease in human corneal epithelial cells under high osmotic pressure conditions.
FIG. 2A is a diagram illustrating an experiment plan according to Example 2 of the present disclosure.
FIG. 2B shows microscope images indicating wound healing identified by cell proliferation and migration as results of an experiment conducted in accordance with the experiment plan of FIG. 2A of the present disclosure, among the effects of the HAPLN1 protein on the relief of dry eye disease in human corneal epithelial cells under high osmotic pressure conditions.
FIG. 2C is a graph showing quantified areas covered with migrated cells shown in the images of FIG. 2B.
FIG. 3A shows images of expression levels of markers of various inflammatory factors by using a Human XL cytokine array (R&D Systems. ARY022B) after treating human corneal epithelial cells with different concentrations of HAPLN1 protein under high osmotic pressure conditions, according to Example 3 of the present disclosure.
FIG. 3B is a graph showing relative expression levels of markers of various inflammatory factors by using a Human XL cytokine array (R&D Systems. ARY022B).
FIG. 4A is a graph showing amounts of CCL-20 secreted in corneal cells after treatment of human corneal epithelial cells with subdivided concentrations of HAPLN1 protein under high osmotic pressure conditions, according to Example 4 of the present disclosure.
FIG. 4B shows a western blot image indicating activity of NFκB as an important transcription factor in the expression of inflammatory factors after treating human corneal epithelial cells with different concentrations of HAPLN1 protein under high osmotic pressure conditions (upper image), and a graph showing quantified relative expression levels thereof (lower image), according to Example 4 of the present disclosure.
FIG. 5A is a diagram illustrating an experimental plan according to Example 5 of the present disclosure to identify expression levels of mucin genes after treating human corneal epithelial cells with different concentrations under high osmotic pressure conditions.
FIG. 5B is a graph showing expression levels of mRNA of MUC4 relatively quantified with reference to the expression levels of mRNA of GAPDH after treating human corneal epithelial cells with different concentrations of HAPLN1 protein under high osmotic pressure conditions, as an experiment conducted according to the experimental plan of FIG. 5A.
FIG. 5C is a graph showing expression levels of mRNA of MUC16 relatively quantified with reference to the expression levels of mRNA of GAPDH after treating human corneal epithelial cells with different concentrations of HAPLN1 protein under high osmotic pressure conditions, as an experiment conducted according to the experimental plan of FIG. 5A.
FIG. 6A shows images indicating expression of MUC5Ac as a representative mucin protein after treating human conjunctiva epithelial cells with different concentrations of HAPLN1 protein under high osmotic pressure conditions, according to Example 6, and FIG. 6B is a graph showing quantified numbers of cells secreting MUC5Ac identified in FIG. 6A.
FIG. 7A illustrates an experimental plan to evaluate efficacy of HAPLN1 in a mouse dry eye model.
FIG. 7B is a diagram for describing sections for score recording of a corneal fluorescein score assay.
FIG. 7C is a view showing a tear volume assay method.
FIG. 8A shows images of eyes indicating corneal damage and degree of alleviation via fluorescent staining in Example 8 of the present disclosure (Day 0, Day 6, and Day 11).
FIG. 8B is a graph showing quantified results of the corneal fluorescein score assay of FIG. 8A (Day 0, Day 6, and Day 11).
FIG. 8C is a graph showing results of the tear volume assay in a mouse dry eye model, i.e., quantified volume of tears absorbed in the Schirmer's test strip (Day 0, Day 6, and Day 11).
FIG. 9A shows images showing conditions of detached corneal epithelial cells on the 16^{th} day from treatment with different concentrations of the composition of the present disclosure.
FIG. 9B is a graph showing quantified numbers of detached corneal epithelial cells in the images of FIG. 9A.
FIG. 10A shows images of goblet cells present in conjunctiva on the 16^{th} day from treatment with different concentrations of the composition of the present disclosure.
FIG. 10B is a graph showing quantified numbers of goblet cells shown in the images of FIG. 10A.
FIG. 11A shows images of expression levels of IL-1β, which is a representative cytokine of inflammatory response, in lacrimal glands on the 16^{th} day from treatment with different concentrations of the composition of the present disclosure, as confirmed by immunohistochemistry.
FIG. 11B is a graph showing quantified expression levels of IL-1β shown in the images of FIG. 11A.
FIG. 12A shows images indicating expression levels of CD45 as an inflammatory cell marker, in lacrimal glands on the 16^{th} day from treatment with different concentrations of the composition of the present disclosure, as confirmed by immunofluorescence (IF).
FIG. 12B is a graph showing quantified expression levels of CD45 shown in the images of FIG. 12A.
FIG. 13A shows images indicating expression levels of CD11b as an inflammatory cell marker, in lacrimal glands on the 16^{th} day from treatment with different concentrations of the composition of the present disclosure, as confirmed by immunofluorescence (IF).
FIG. 13B is a graph showing quantified expression levels of CD11b shown in the images of FIG. 13A.
FIG. 14 is a diagram showing an experimental plan for inducing a rabbit dry eye model by using benzalkonium chloride and evaluating efficacy of HAPLN1 thereon.
FIG. 15A shows images of eyes before conducting a corneal fluorescein score assay for indicating the degree of corneal damage of rabbit eyes.
FIG. 15B is a graph showing tear film break up time (TFBUT) taken for the tear film to break down depending on concentrations of the composition of the present disclosure in a rabbit dry eye model induced by using benzalkonium chloride.
FIG. 15C is a graph showing quantified results of a corneal fluorescein score (CFS) assay on Day 0, Day 5, Day 10, and Day 15 for indicating the degree of corneal damage of eyes in the images of FIG. 15A depending on concentrations of the composition of the present disclosure.
FIG. 15D is a graph showing quantified tear volumes on Day 0, Day 5, Day 10, and Day 15 calculated based on volume of tears absorbed to the Schirmer test strip of FIG. 15A according to concentrations of the composition of the present disclosure.
FIG. 16A shows images indicating degrees of detachment of corneal epithelial cells on the 16^{th} day from treatment with different concentrations of the composition of the present disclosure in a rabbit dry eye model.
FIG. 16B is a graph showing quantified numbers of corneal epithelial cells detached in each case of FIG. 16A.
FIG. 17A shows images indicating conditions of goblet cells present in conjunctiva on the 16^{th} day from treatment with different conditions of the composition of the present disclosure in a rabbit dry eye model.
FIG. 17B is a graph showing quantified numbers of goblet cells in each case of FIG. 17A.

### Best Mode

An aspect of the present disclosure relates to a pharmaceutical composition for prevention or treatment of dry eye disease, including hyaluronan and proteoglycan link protein 1 (HAPLN1) or a gene encoding HAPLN 1 as an active ingredient.

Furthermore, according to an embodiment of the present disclosure, the pharmaceutical composition of the present disclosure may act to induce wound healing in cells in the eye. This is because the composition of the present disclosure promotes cell proliferation and migration to the wound.

According to an embodiment of the present disclosure, the pharmaceutical composition of the present disclosure may act to reduce expression of inflammatory factors of a corneal epithelial cell. Examples of the inflammatory factor may be Interleukins, CCL/CXCL family, Chitinase 3-like 1, pNFkB, or the like.

Also, according to an embodiment of the present disclosure, the pharmaceutical composition of the present disclosure may act to increase the expression level of mucin. This is because the composition of the present disclosure promotes the expression of mRNA of various mucin families and galectin-3.

In the specification of the present disclosure, the "rhHAPLN1" is an abbreviation for recombinant human HAPLN1 indicating recombinant human HAPLN1.

In some embodiments, the pharmaceutical composition of the present disclosure may generally include molecules and a pharmaceutically acceptable carrier. As used herein, the term "pharmaceutically acceptable carrier" includes saline solution, a solvent, a dispersion medium, a coating agent, an antibacterial agent, an antifungal agent, an isotonic agent, and an absorption delaying agent which are compatible with pharmaceutical administration. Supplementary active compounds may also be included in the composition. In other words, the pharmaceutical composition of the present disclosure may further include a pharmaceutical additive selected from the group consisting of pharmaceutically acceptable carriers, diluents, binders, disintegrants, lubricants, and any combinations thereof.

The pharmaceutical composition may be formulated to be compatible with intended route of administration. Preferably, the composition of the present disclosure may be a formulation selected from the group consisting of eye drops, artificial tears, ophthalmic ointments, tablets, pills, capsules, troches, inhalants, injections, patches, and suppositories.

Examples of the route of administration include parenteral, for example, intravenous, intradermal, subcutaneous, oral (e.g., inhalation), transdermal (topical), transmucosal, and rectal administration. Preferably, parenteral administration may be used.

Solutions or suspensions used for parenteral, intradermal, or subcutaneous application may include following ingredients: sterile diluents such as distilled water for injection, saline solution, fixing oil, polyethylene glycol, glycerin, propylene glycol, or other synthetic solvents; antibacterial agents such as benzyl alcohol or methyl paraben; antioxidants such as ascorbic acid and sodium bisulfite; and chelating agents such as ethylenediaminetetraacetic acid; buffers such as acetate, citrate, and phosphate; and tonicity-adjusting agents such as sodium hydroxide or dextrose. The pH may be adjusted by acid or base such as hydrochloric acid or sodium hydroxide. Parenteral preparations may be placed in ampoules, disposable syringes, or multiple-dose vials made of glass or plastic.

Pharmaceutical compositions suitable for injectable use include a sterile aqueous solution (if water-soluble) or dispersions and sterile powders for extemporaneous preparation of sterile injectable solutions or dispersions. For intravenous administration, suitable carriers include physiological saline, bacteriostatic water, or phosphate buffered saline (PBS). In all cases, the composition should be sterile and should be fluid to the extent that easy injectability exists. The pharmaceutical formulations should be stable under the conditions of manufacture and storage and should be preserved against the contaminating action of microorganisms such as bacteria and fungi. The carrier may be a solvent or dispersion medium containing, for example, water, ethanol, polyol (such as glycerol, propylene glycol, and liquid polyethylene glycol), and a suitable mixture thereof. For example, proper fluidity may be maintained by using coating such as lecithin, by maintaining required particle size in the case of dispersion, or by using a surfactant. Prevention of the action of microorganisms may be achieved by various antibacterial and antifungal agents, such as paraben, chlorobutanol, phenol, ascorbic acid, and thimerosal. In many cases, it will be preferable to add isotonic agents, such as sugar, polyalcohol such as mannitol, sorbitol, or sodium chloride to the composition. Prolonged absorption of an injectable compositions may be obtained by adding an agent that delays absorption, for example, aluminum monostearate and gelatin, to the composition.

Sterile injectable solutions may be prepared by incorporating an active ingredient in a required amount into an appropriate solvent with one or a combination of the listed ingredients, as required, followed by filtration sterilization. In general, dispersions are prepared by incorporating the active compound into a sterile vehicle including a basic dispersion medium and other required ingredients listed above. In the case of sterile powders for the preparation of sterile injectable solutions, the preferred preparation methods are vacuum drying and freeze drying which yield powder of the active ingredient and any additional desired ingredient from a previously sterile-filtered solution thereof.

A composition for oral administration generally includes an inert diluent or an edible carrier. For the purpose of oral therapeutic administration, the active compound may be incorporated with an excipient and may be used in the form of tablet, troche, capsule, for example, gelatin capsule. Compositions for oral administration may also be prepared by using a fluid carrier for use as mouth rinse. Pharmaceutically suitable binders and/or adjuvant substances may be included as parts of the composition. Tablets, pills, capsules, troches, and the like may include any one of the following ingredients or compounds having similar properties: a binder, e.g., microcrystalline cellulose, tragacanth gum, or gelatin; an excipient such as starch or lactose, a disintegrant such as alginic acid, Primogel, or corn starch; a lubricant such as magnesium stearate or sterotes; a glidant such as colloidal silicon dioxide; a sweetener such as sucrose or saccharin; or a flavor such as peppermint, methyl salicylate, or orange flavor.

A compound for administration by inhalation is delivered in the form of an aerosol spray from a pressure vessel or dispenser including a suitable propellant, e.g., gas such as carbon dioxide or a nebulizer.

Systemic administration may also be performed by transmucosal or transdermal method. In the case of transmucosal or transdermal administration, a penetrating agent suitable for a barrier to be penetrated is used. In general, the penetrating agent is well known in the art and includes, for example, an agent for transmucosal administration, a cleaner, a bile salt, and a fusidic acid derivative. Transmucosal administration may be performed by using a nasal spray or suppository. An active compound for transdermal administration may be formulation as ointments, salves, gels, or creams as well known in the art.

The compounds may be prepared in the form of suppositories (e.g., with common suppository base such as cocoa butter and other glyceride) or retention enemas for rectal delivery.

Data obtained from cell culture assays and animal studies may be used to formulate various dosages for use in humans. The dosage of the compound is preferably within a range of circulating concentrations that include ED₅₀ with little or to toxicity. The dosage may vary within this range according to administration form and route of administration.

A therapeutically effective amount (i.e., effective dose) of the composition of the present disclosure varies according to the conditions of a selected patient. For example, a single dose of about 1 pg to 1000 mg may be administered. In some embodiments, the composition may be administered in an amount of 10, 30, 100, or 1000 pg, or 10, 30, 100, or 1000 ng, or 10, 30, 100, or 1000 µg, or 10, 30, 100 or 1000 mg.

In some embodiments, 1 ng/ml to 100 µg/ml, preferably, 3 ng/ml to 50 µg/ml, more preferably, 5 ng/ml to 500 ng/ml, particularly preferably, 10 ng/ml to 200 ng/ml of the composition may be administered. The composition may be administered from once a week to more than once a week including once every other day. Those skilled in the art will recognize that certain factors, which may affect dose and timing required to effectively treat an individual, may include severity of disease or disorder, previous treatment, general health state and/or age and other pre-existing conditions of the individual, but are not limited thereto. Furthermore, treatment of an individual with a therapeutically effective amount of molecules of the present disclosure may include a single treatment or, preferably, a series of treatments.

The dose of the composition of the present disclosure is within a range of 5 mg/kg/week to 500 mg/kg/week, for example, 5 mg/kg/week, 10 mg/kg/week, 15 mg/kg/week, 20 mg/kg/week, 25 mg/kg/week, 30 mg/kg/week, 35 mg/kg/week, 40 mg/kg/week, 45 mg/kg/week, 50 mg/kg/week, 55 mg/kg/week, 60 mg/kg/week, 65 mg/kg/week, 70 mg/kg/week, 75 mg/kg/week, 80 mg/kg/week, 85 mg/kg/week, 90 mg/kg/week, 95 mg/kg/week, 100 mg/kg/week, 150 mg/kg/week, 200 mg/kg/week, 250 mg/kg/week, 300 mg/kg/week, 350 mg/kg/week, 400 mg/kg/week, 450 mg/kg/week, and 500 mg/kg/week depending on conditions of a patient. In some embodiments, a dose of dual functional molecules according to the present disclosure is within a range of 10 mg/kg/week to 200 mg/kg/week, 20 mg/kg/week to 150 mg/kg/week, or 25 mg/kg/week to 100 mg/kg/week. In some embodiments, the composition of the present disclosure is administered 1× times a week for 2 weeks to 6 months, for example, for 2 weeks, 3 weeks, 4 weeks, 5 weeks, 6 weeks, 7 weeks, 8 weeks, 9 weeks, 10 weeks, 11 weeks, 12 weeks, 13 weeks, 26 weeks, 6 months, 8 months, 10 months, or over 1 year. In another embodiment, the composition of the present disclosure is administered 2x times a week. In another embodiment, the composition of the present disclosure is administered every other week.

The composition of the present disclosure may be formulated as a pharmaceutical composition including a pharmacologically effective amount of HAPLN1 protein molecules and a pharmaceutically acceptable carrier. The pharmacologically or therapeutically effective amount refers to an amount effective to obtain intended pharmacological, therapeutic, or prophylactic results. The term "pharmacologically effective amount" and "therapeutically effective amount", or simply "effective amount" refers to an amount of dual functional molecules effective to obtain intended pharmacological, therapeutic, or prophylactic results. For example, if a decrease in a measurable parameter associated with a disease or disorder by 20 % or more by a given clinical treatment is considered effective, the therapeutically effective amount of a drug to treat the disease or disorder refers to an amount require to reduce the parameter by at least 20 %.

The pharmaceutical composition of the present disclosure that is properly formulated may be administered by any routes well known in the art such as parenteral routes including intravenous, intramuscular, intraperitoneal, subcutaneous, transdermal, respiratory (aerosol), rectal, vaginal, and topical (including buccal and sublingual). In some embodiments, the pharmaceutical composition may be administered intravenously or parenteral infusion or injection.

In general, suitable daily dosage units of molecules are in a range of 0.001 to 0.25 mg per body weight (kg), 0.01 to 20 µg per body weight (kg), 0.01 to 10 µg per body weight (kg), 0.10 to 5 µg per body weight (kg), or 0.1 to 2.5 µg per body weight (kg) of a recipient. The pharmaceutical composition including the molecules may be administered once a day. However, the therapeutic agent may also be administered in dosage units including 2, 3, 4, 5, 6, or more sub-doses administered at appropriate intervals through the day. Dosage units may be prepared as single dose over several days, for example, by using a sustained releasing formulation well known in the art that provides the molecules continuously and consistently. The sustained release formulation is well known in the art. In this embodiment, the dosage unit includes multiples corresponding to daily dose.

The pharmaceutical composition may be included in a kit, container, pack, or dispenser together with administration instructions.

Another aspect of the present disclosure provides a method for preventing or treating dry eye disease, the method including administering hyaluronan and proteoglycan link protein 1 (HAPLN1) as an active ingredient to an individual in need thereof.

The present disclosure provides both preventive and therapeutic methods for treating an individual with (or sensitive to) dry eye disease.

As used herein, the terms "treatment" or "treating" are defined as applying or administering a therapeutic agent (for example, molecules of the present disclosure) or applying or administering isolated tissues or cell lines to a patient for the purpose of treating, curing, alleviating, relieving, modifying, ameliorating, or influencing a disease or disorder, symptoms of the disease or disorder, or causes of the disease of disorder, wherein the patient had the disease or disorder, symptoms of the disease or disorder, or causes of the disease of disorder.

Another aspect of the present disclosure provides a method for preventing the disease or disorder described above in an individual by administering the composition of the present disclosure to the individual. Individuals at the risk of developing the disease may be identified, for example, by one or a combination of diagnostic or prognostic analyses described herein.

Another aspect of the present disclosure provides a method for treating or suppressing dry eye disease by treating an individual with a composition including hyaluronan and proteoglycan link protein 1 (HAPLN1) or a gene encoding the same as an active ingredient, i.e., a method of modifying development of symptoms of dry eye disease. These methods may be performed in vitro or, alternatively, in vivo (for example, by administering the composition of the present disclosure to an individual).

Another aspect of the present disclosure provides a use of hyaluronan and proteoglycan link protein 1 (HAPLN1) or a gene encoding the same for preparation of a composition for preventing or treating dry eye disease.

Furthermore, another aspect of the present disclosure provides a use of a composition including hyaluronan and proteoglycan link protein 1 (HAPLN1) as an active ingredient for preventing or treating dry eye disease.

### Mode for Invention

Hereinafter, the present disclosure will be described in more detail with reference to the following examples. However, the following examples are merely presented to exemplify the present disclosure, and the scope of the present disclosure is not limited thereto.

### [Preparation Example 1]

### Preparation of Hyaluronan and Proteoglycan Link Protein 1 (HAPLN1)

A vector including a polynucleotide encoding human HAPLN1 protein having an amino acid sequence of SEQ ID NO: 1 was inserted into CHO-K1 cells to construct a CHO-K1 cell line that produces recombinant human HAPLN1 protein.

The amino acid sequence of SEQ ID NO: 1 is as follows:

MKSLLLLVLISICWADHLSDNYTLDHDRAIHIQAENGPHLLVEAEQAKVFSHRG
GNVTLPCKFYRDPTAFGSGIHKIRIKWTKLTSDYLKEVDVFVSMGYHKKTYGGYQGRV
FLKGGSDSDASLVITDLTLEDYGRYKCEVIEGLEDDTVVVALDLQGVVFPYFPRLGRYN

LNFHEAQQACLDQDAVIASFDQLYDAWRGGLDWCNAGWLSDGSVQYPITKPREPCG
GQNTVPGVRNYGFWDKDKSRYDVFCFTSNFNGRFYYLIHPTKLTYDEAVQACLNDGA
QIAKVGQIFAAWKILGYDRCDAGWLADGSVRYPISRPRRRCSPTEAAVRFVGFPDKKH
KLYGVYCFRAYN

A cell line having excellent protein production and quality was selected as a master cell bank (MCB). The MCB was subcultured and inoculated onto a Hyperforma SUB 250 L bioreactor manufactured by Thermo in a concentration of 0.40±0.05×10⁶ cells/mL and fed-batch cultured. As a basic medium, a mixture of 22.36 g of ActiPro^{™} medium, 0.5846 g of glutamine, 10.00 g of HT Supplement (Thermo Fisher Scientific), 4.29 g of 10 N NaOH, and 1.80 g of NaHCOs was used. An incubation temperature was set to 36.5°C, a dissolved oxygen (DO) was set to 40.0%, and a pH was set to 7.00±0.20. As a pH regulating solution, 1 M sodium carbonate monohydrate was used. As a feeding medium (FM), FM020a including 181.04 g of HyClone^{™} Cell Boost 7a and 12.28 g of 10 N NaOH and FM020b including 94.60 g of HyClone^{™} Cell Boost 7b and 105.93 g of 10 N NaOH were used.

Recombinant human HAPLN1 (rhHAPLN1) protein was recovered (Harvest and Clarification) from the cells incubated as described above and isolated and purified via processes such as Ultrafiltration/Diafiltration 1 (UF/DF1), Anion Exchange Chromatography, Solvent/Detergent (S/D) virus inactivation, Cation Exchange Chromatography, Mixed-mode Chromatography (MMC), Hydrophobic Interaction Chromatography, Ultrafiltration/Diafiltration 2 (UF/DF2), and Intermediate Depth Filtration, (Int. DF). In the following examples, the composition of the present disclosure as described above was used.

### [Preparation Example 2]

### Preparation of Composition of Present Disclosure including HAPLN1 Protein

The HAPLN1 protein purified in Preparation Example 1 was stabilized in a 20 mM acetic acid buffer, 8% (w/v) sucrose, and 0.04% (w/v) PS80 at a pH of 5.0 to prepare a composition of the present disclosure including the HAPLN1 protein as a main active ingredient.

### [Preparation Example 3]

### Preparation of Composition of Present Disclosure including Expression Vector Containing Gene Encoding HAPLN1 Protein

A composition including an expression vector containing a gene encoding HAPLN1 protein was prepared by using a jetPRIME transfection reagent kit (manufactured by PolyPlus, Model No. 114-15). More specifically, a plasmid vector designed to express Human HAPLN1 (hereinafter, referred to as "Human HAPLN1 ORF Clone") was prepared by using RC209274 manufactured by OriGene. The vector may express the gene, transported by the vector, in nuclei of host cells.

For reference, the jetPRIME transfection reagent kit consists of jetPRIME buffer and jetPRIME Transfection reagent, and the jetPRIME buffer is used to dilute the plasmid vector transported to the host cells, and the jetPRIME transfection reagent traps the plasmid vector in the form of a vesicle (liposome) and allows the vector to penetrate into host cells.

The Human HAPLN1 ORF Clone was diluted in the jetPRIME buffer to prepare the composition including the expression vector containing the HAPLN1 protein-encoding gene according to the present disclosure.

ThejetPRIME transfection reagent was added thereto and allowed to stand for 10 minutes to trap the plasmid vector in the form of a vesicle (liposome) and then the plamid vector was dropped on the cells being cultured. After incubating the cells for 48 hours or more, an increase in expression of the human HAPLN1 gene was identified by real-time qPCR and increase, and an increase in expression of the human HAPLN1 protein was identified by Western blot.

### [Example 1]

### Confirmation of Effect of Composition (HAPLN1 protein) of Present Disclosure on Relief of Dry Eye Disease (Cell Viability) in Human Corneal Epithelial Cell (HCEC) under High Osmotic Pressure Conditions

In most dry eye diseases, tear decreases in the aqueous layer in an eyeball due to lacrimal hyposecretion or hyperevaporation of tear. In this case, osmotic pressure increases because water decreases while concentrations of proteins, lipids, carbohydrates, etc. contained in tear are constantly maintained. Based on this principle, in cellular level studies, NaCl is added to a cell culture medium to control the osmotic pressure, establish a dry eye disease model, and evaluate efficacy of a drug. Thus, in the present embodiment, by using a case with an osmotic pressure of 314 mOsM as a normal control and a case with a relatively high osmotic pressure of 450 mOsM as a dry eye disease model, effects of HAPLN1 protein on the relief of dry eye disease in human corneal epithelial cells under the high osmotic pressure conditions were verified. This is related to cell viability of human corneal epithelial cells.

In this experiments, a cell counting kit-8 (CCK-8), which allows a sensitive colorimetric assay used to measure cell viability and cytotoxicity, was used. That is, addition of WST-8, which is a highly water-soluble tetrazolium salt, to cells lowers cellular dehydrogenase activity to produce an orange-colored formazan dye in the cells. Because the amount of the dye is related to the number of viable cells, a larger amount of the dye indicates more viable cells.

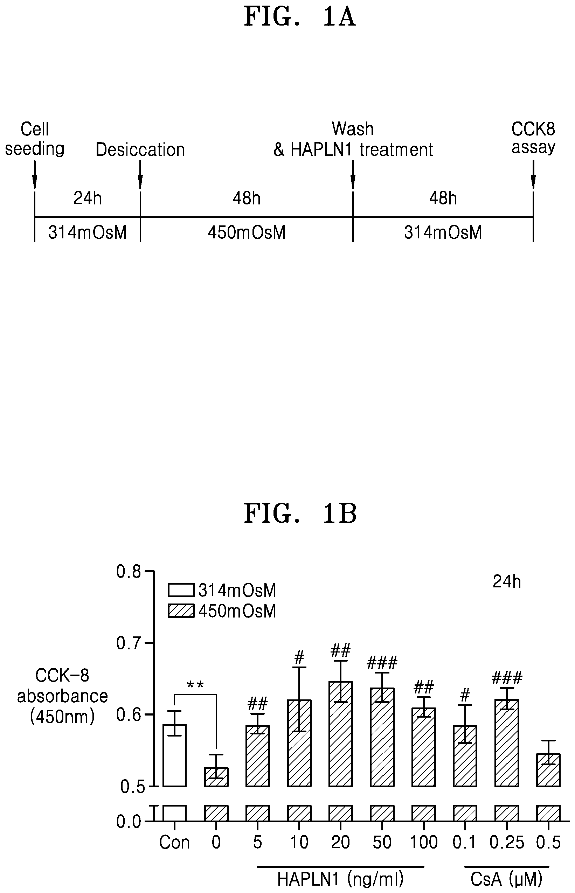

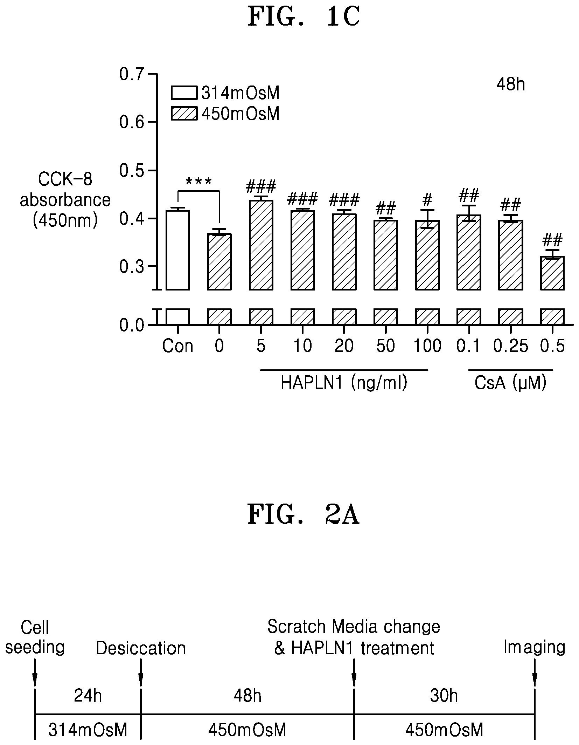

That is, the human corneal epithelial cells were seeded onto a Petri dish and incubated for 24 hours under low osmotic pressure conditions (314 mOsM). Subsequently, the cells were incubated under high osmotic pressure conditions (450 mOsM) for 48 hours, washed with a PBS buffer, and treated with different concentrations of the composition including HAPLN1 protein of the present disclosure. Thereafter, the cells were additionally incubated for 24 hours and 48 hours, and then assayed by using the CCK-8 (FIG. 1A). FIGS. 1B and 1C are graphs showing results of an experiment conducted in accordance with the experiment plan of FIG. 1A indicating cell viability after 24 hours (FIG. 1B) and 48 hours (FIG. 1C) from administration of the composition according to the present disclosure among the effects of the HAPLN1 protein on the relief of dry eye disease in the human corneal epithelial cells under the high osmotic pressure conditions. Based thereon, as a result of treating the cells with 5 to 100 ng/ml of the HAPLN1 protein contained in the composition according to the present disclosure and incubating the cells for 24 hours or 48 hours, CCK-8 absorbances (450nm) not less than or similar to that of a normal cell group (low osmotic pressure control) were observed in all concentrations.

For reference, in the graphs, CsA refers to Cyclosporin A, a main ingredient of Restasis as a control drug and the composition of the present disclosure, surprisingly, showed numerical values higher than 0.1 to 0.5 µM of Cyclosporin A regardless of the concentration thereof.

Based on these results, although the cell viability is decreased by treatment with 450 mOsM, it was confirmed that the cell viability increased in the group treated with the composition including the HAPLN1 protein according to the present disclosure, and thus it was proved that the HAPLN1 protein contributes to an increase in cell viability. That is, it was confirmed that the composition including the HAPLN1 protein according to the present disclosure may relieve dry eye disease in human corneal epithelial cells.

### [Example 2]

### Confirmation of Effect of Composition (HAPLN1 protein) of Present Disclosure on Relief of Dry Eye Disease (Cell Proliferation and Migration) in Human Corneal Epithelial Cell (HCEC) under High Osmotic Pressure Conditions

In relation to dry eye disease, it was verified, via cell proliferation and migration phenomena, that the HAPLN1 protein-containing composition of the present disclosure induces wound healing of cells as one of the effects on the relief of dry eye disease.

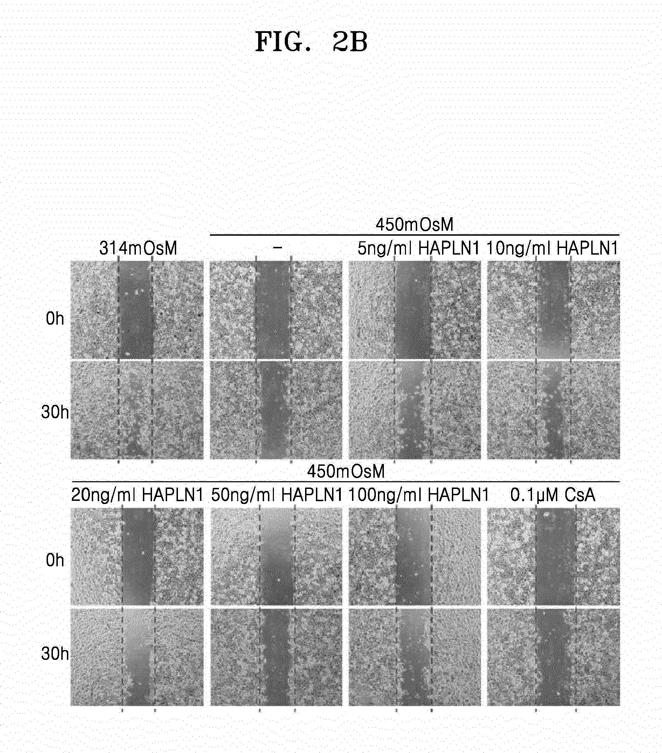

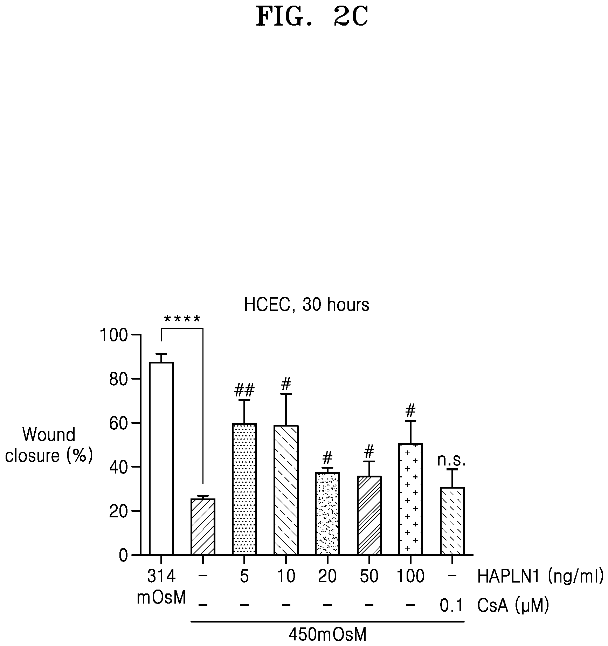

The human corneal epithelial cells were seeded onto 24 wells and incubated for 24 hours under low osmotic pressure conditions (314 mOsM). Then, after replacing the culture medium with a high osmotic pressure medium (450 mOsM), the cells were incubated for 48 hours. Then, the surface of the Petri dish was vertically scratched and washed twice, and then the culture medium was replaced with the high osmotic pressure medium again and simultaneously the cells were treated with different concentrations of the HAPLN1 protein-containing composition of the present disclosure (FIG. 2A). A reduction in the scratched area was confirmed by obtaining photographs using a microscope at a time of scratching (0 h) and after 30 hours (FIG. 2B). This was imaged and a ratio of an area filled with cells to an initial area was identified by using imaged (FIG. 2C).

FIG. 2B shows microscopic images indicating wound healing identified by cell proliferation and migration as results of an experiment conducted in accordance with the experiment plan of FIG. 2A according to the present disclosure among the effects of the HAPLN1 protein on the relief of dry eye disease in the human corneal epithelial cells under the high osmotic pressure conditions. FIG. 2C is a graph showing quantified areas covered with migrated cells shown in the images of FIG. 2B.

According to FIGS. 2B and 2C, in the case of treatment with 5 to 100ng/ml off the HAPLN1 protein, cell proliferation and migration phenomena of scratched surfaces were observed over the entire area. Particularly, cell proliferation and migration of covering about 60 % of the scratched surface were observed even under high osmotic pressure conditions in the case of treatment with 5 and 10 ng/ml of HAPLN1, and this result indicates very high efficacy upon comparison with about 82 % of normal cells. Also, it was confirmed that similar efficacy was significantly observed in other concentrations. For reference, in the graphs, CsA refers to Cyclosporin A, a main ingredient of Restasis that was used as a control drug, and the composition according to the present disclosure, surprisingly, showed numerical values higher than 0.1 µM of Cyclosporin A regardless of the concentration thereof.

### [Example 3]

### Confirmation 2 of Effect of Composition (HAPLN1 protein) of Present Disclosure on Relief of Inflammation in Human Corneal Epithelial Cell (HCEC) under High Osmotic Pressure Conditions

As one of the effects the HAPLN1 protein-containing composition of the present disclosure on the relief of dry eye disease, an effect on the relief of inflammation was verified by using Human XL cytokine array (R&D Systems. ARY022B) based on expression levels of inflammatory factors (Interleukins, CCL/CXCL family, and Chitinase 2-like 1).

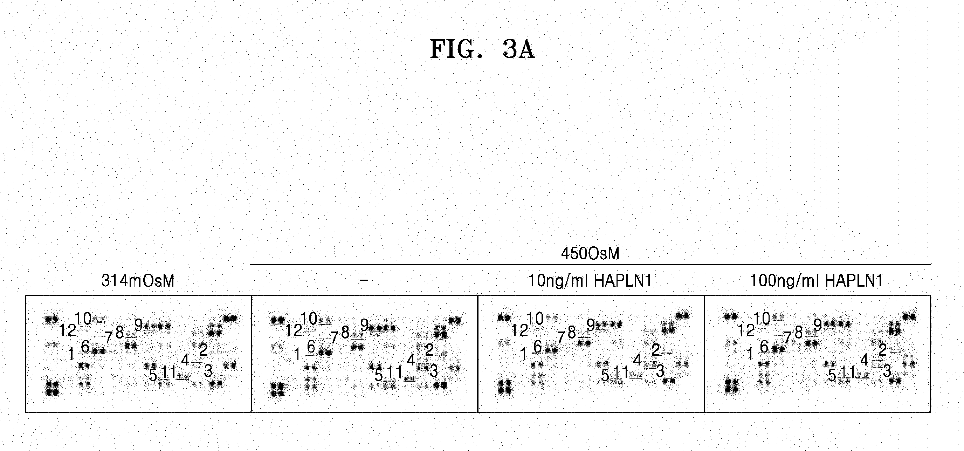

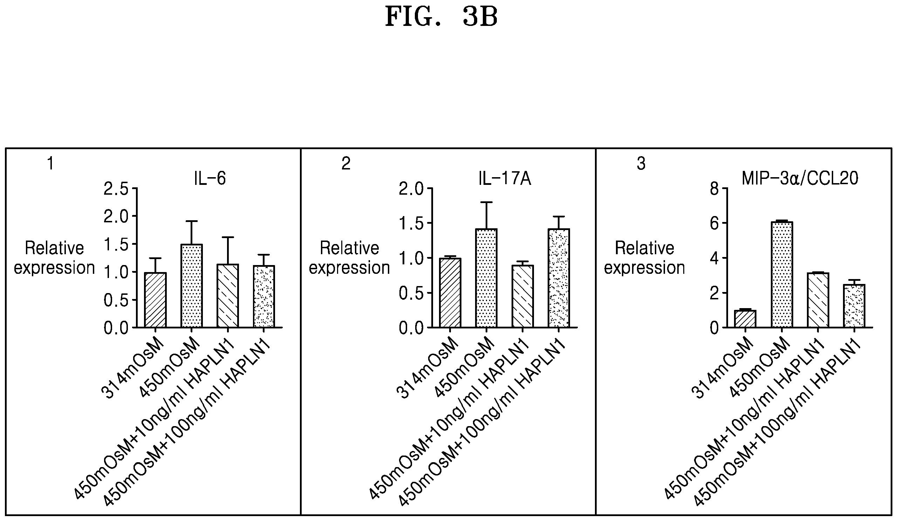

First, the human corneal epithelial cells after 4^{th} passage were seeded onto 6 wells and incubated for 24 hours under low osmotic pressure conditions (314 mOsM). Subsequently, the cells were incubated under high osmotic pressure conditions (450 mOsM) for 48 hours and treated with different concentrations (i.e., 10 µg/ml HAPLN 1 µl to 1 ml media --> working concentration of 10 ng/ml) of the composition according to the present disclosure. Then, the cells were further incubated for 24 hours, and the culture broth was recovered therefrom, followed by an assay on the expression level of each marker by using Human XL cytokine array (R&D Systems. ARY022B). Then, the results are shown in FIG. 3A. According to each of the graphs of FIG. 3B illustrating relative expression levels of the markers of the inflammatory factors in the different concentrations of the HAPLN1 protein, it was confirmed that secretion of inflammatory factors such as IL-6, IL-17A, CCL20, CXCL10, CCL5, CD54, and CD176 increased under the high osmotic pressure conditions (450 mOsM). However, in the composition of the present disclosure, it was confirmed that the expression levels of the inflammatory factors of groups treated with 10 to 100 ng/ml of the HAPLN1 protein were lower than that of untreated groups, i.e., inflammation was relieved in the cells. In other words, when the expression level of the factor in normal cells was considered as 1.0, the expression level of abnormal groups increased to 1 or more, but the expression levels of various inflammatory factors decreased to the level of the normal group or less by treatment of the composition including the rhHAPLN1 recombinant protein according to the present disclosure. Particularly, the expression levels of the inflammatory factors were significantly high in the MIP-3a/CCL20 and IP-10/CXCL10 under high osmotic pressure conditions (450 mOsM). However, it was confirmed that the expression levels of various inflammatory factors IL-6, IL-17A, MIP-3a/CCL20 and IP-10/CXCL10, RANTES5/CCL5, ICAM-1/CD54, and Fas ligands/CD178 may be reduced by treatment with the HAPLN1 protein-containing composition of the present disclosure, and it was confirmed that the expression levels of IL-17A, Fas ligand/CD178, FGF-7, FGF-19, Angiogenin, RBP-4, and Chitinase 3-like 1 were reduced to the levels of normal cells.

Based on the results, it was verified that the HAPLN1 protein-containing composition of the present disclosure relieved inflammations of the human corneal epithelial cells as an effect on the relief of dry eye disease.

### [Example 4]

### Confirmation 2 of Effect of Composition (HAPLN1 protein) of Present Disclosure on Relief of Inflammation in Human Corneal Epithelial Cell (HCEC) under High Osmotic Pressure Conditions

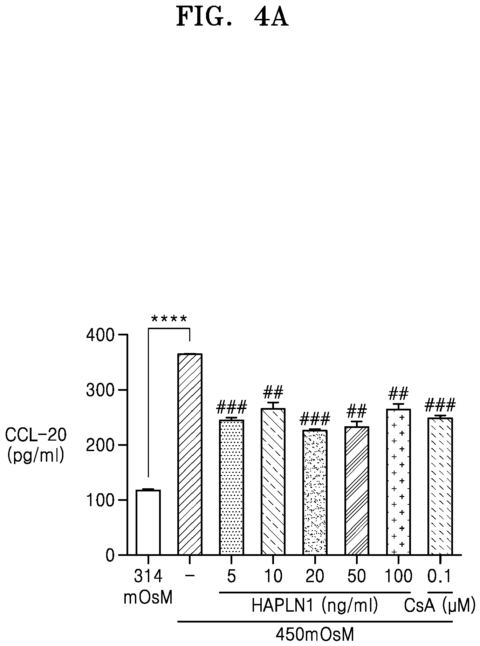

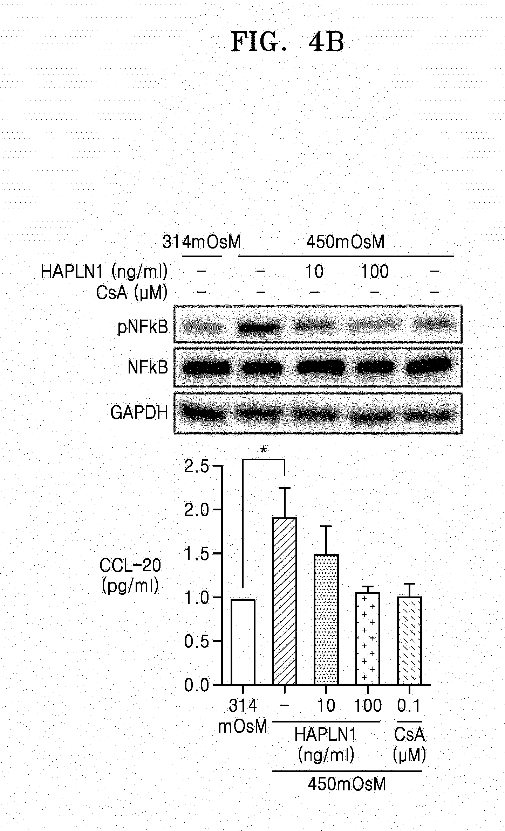

In order to reconfirm the effect of the HAPLN1 protein-containing composition according to the present disclosure on the expression of CCL-20, which showed the greatest variation in Example 3, the concentration was subdivided and an ELISA experiment was performed (FIG. 4A). In addition, the activity of NFκB that is an important transcription factor in expression of the inflammatory factors was identified by Western blot analysis (FIG. 4B). Preparation of the experiment was performed under the same conditions of those of Cytokine array performed as described above and the ELISA was performed according to the manufacturer's instructions.

In the results using the ELISA experimental method, similar to the results of the previously performed Cytokine array, it was confirmed that secretion of the inflammatory factor CCL20 increased by almost 4 times under the high osmotic pressure conditions (450 mOsM) (FIG. 4A). However, in the case of 5, 10, 20, 50, and 100 ng/ml of the HAPLN1 protein-containing composition of the present disclosure, it was confirmed that the expression levels of the inflammatory factor were relatively lower than those of the HAPLN1 protein-untreated groups, i.e., that cellular inflammation was significantly reduced.

In addition, it was confirmed that phosphorylation of NFκB, which is a transcription factor playing an important role in regulation of expression of the inflammatory factors, was reduced by the HAPLN1 protein-containing composition of the present disclosure (FIG. 4B).

Based on the results, it was verified again that the HAPLN1 protein-containing composition according to the present disclosure relieved inflammation in the human corneal epithelial cells by suppressing the activity of NFκB as an effect on the relief of dry eye disease.

### [Example 5]

### Confirmation 2 of Effect of Composition (HAPLN1 protein) of Present Disclosure on Relief of Dry Eye Disease (increase in Mucin) in Human Corneal Epithelial Cell (HCEC) under High Osmotic Pressure Conditions in In Vitro Model

Mucin is a sticky liquid mainly including mucopolysaccharide and glycoprotein. Dry eye disease causes a decrease in goblet cells which is closely related to a decrease in secretion of gel-forming mucin as an important marker of dry eye disease. There are two types of mucin. Group 1 is membrane associated mucin (MAM) including MUC3A/B, MUC4, MUC11/12, MUC16, and MUC17, and Group 2 is secreted mucin including MUC2, MUC5AC, MUC5B, MUC6, and MUC19. The mucins belonging to the MAM of Group 1 contribute to formation of mucus gel at relatively lower levels, and the secreted mucin of Group 2 plays an important role in gel-forming shape. The secreted mucins of Group 2 are polymers or macromolecules mainly synthesized and secreted from particular cells such as goblet cells, and expression of MUC5AC was confirmed in conjunctival cells. Expression of Glycocalyx mucins is known to be closely related to dry eye disease (doi:https://doi.org/10.1167/iovs.17-23756).

This example was designed to identify whether the HAPLN1 protein-containing composition of the present disclosure increases both MAM and the secreted mucin and to verify the effect of the composition of the present disclosure on the relief of dry eye disease.

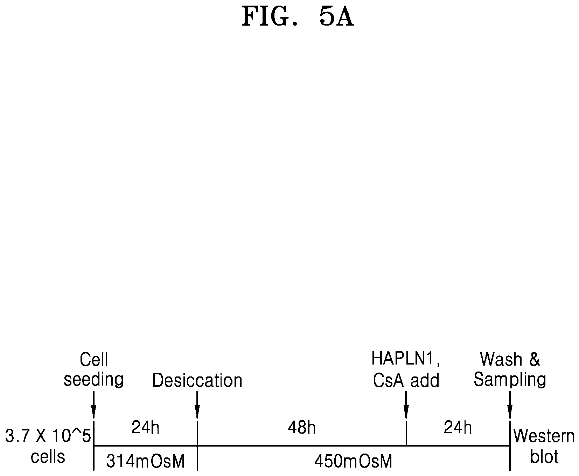

First, the human corneal epithelial cells (HCECs, 5^{th} passage) were seeded onto 6 wells at a density of a corneal epithelial cell basal medium. After 24 hours, the HCECs were pre-treated with a medium with different osmotic pressures (314 and 450 mOsM). After 48 hours of incubation, the cells were treated with the composition of the present disclosure having different concentrations of rhHAPLN1 (0, 5, 10, 20, 50, 100, and 200 ng/ml) and Cyclosporine A (0.1 µM), as a control drug, for 24 hours. Then, the cells were washed and sampled to perform RT-qPCR (FIG. 5A).

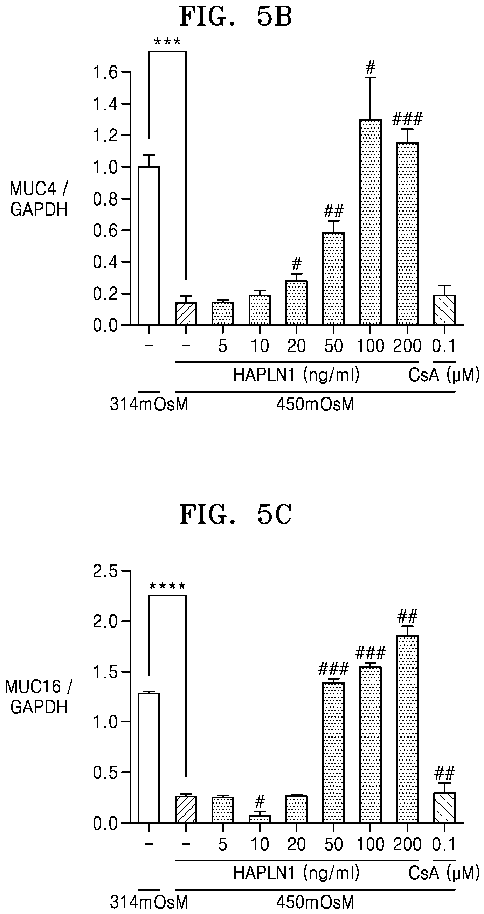

Total mRNA was purified from the HCECs by using a Qiagen kit. A cDNA was synthesized by using the iScript cDNA synthesis kit. Subsequently, RT-qPCR was performed and result data was expressed as relative expression levels (fold) (FIGS. 5B to 5C).

Based on the results, although the expression levels of MUC4 and MUC16 decreased in the dry eye disease-induced model compared to the normal group, the expression level of MUC4 significantly increased when treated with the composition of the present disclosure including 10 to 200 ng/ml, particularly, 50 to 200 ng/ml, of HAPLN1 protein, compared to the untreated group (FIG. 5B). Also, the expression level of MUC4 also significantly increased when treated with the composition of the present disclosure including 20 to 200 ng/ml, particularly, 50 to 200 ng/ml, of HAPLN1 protein, compared to the untreated group (FIG. 5C).

Based on the results, it was proved that the HAPLN1 protein-containing composition of the present disclosure increases expression of various mucins in the human corneal epithelial cells as one of the effects on the relief of dry eye disease.

### [Example 6]

### Confirmation 2 of Effect of Composition (HAPLN1 protein) of Present Disclosure on Relief of Dry Eye Disease (increase in Mucin) in Human Corneal Epithelial Cell (HCEC) under High Osmotic Pressure Conditions in In Vitro Model

In relation to expression of mucin, secretion of MUC5AC in goblet cells is an important condition for maintaining the tear film. That is, it is well known that the goblet cells secrete Mucin Glycocalix that is essential to maintain the tear film and the number of the goblet cells decrease in dry eyes. MUC5AC is also known as a marker of the goblet cells.

First, human conjunctiva epithelial cells (HConEpiC) obtained after 4^{th} passage of culture were seeded onto 24 wells and incubated for 24 hours, and then treated with high osmotic pressure of 450 mOsM simultaneously with the composition of the present disclosure having various concentrations (0, 5, 10, 20, 50, 100, and 200 ng/ml) of the HAPLN1 protein or Cyclosporine A (0.1 µM). After 72 hours of incubation, the cells were immobilized with 4 % paraformaldehyde for 30 minutes and washed with a buffer. Subsequently, fluorescence staining was performed by using Anti-MUC5AC (DAPI labels the nuclei).

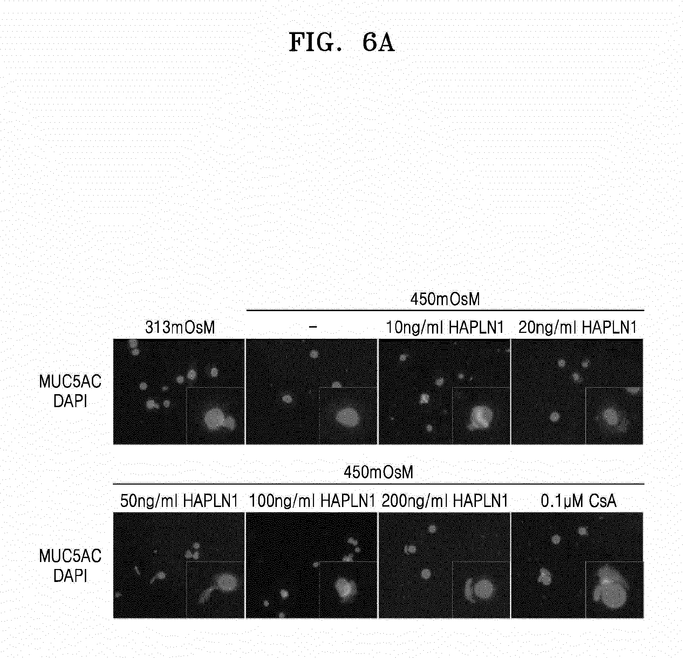

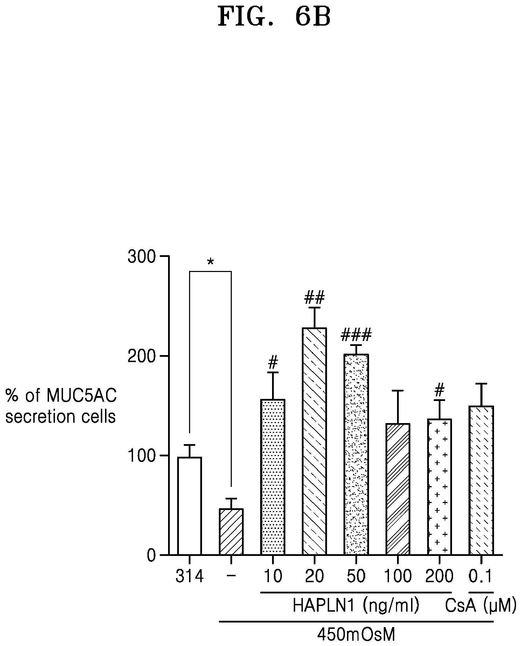

As a result of the experiment, FIG. 6A shows fluorescent images indicating protein expression and secretion of MUC5AC. FIG. 6B is a graph showing quantified numbers of cells secreting MUC5AC as shown in FIG. 6A. Based thereon, in al the case of treatment with different concentrations (10, 20, 50, 100, and 200 ng/ml) of the HAPLN1 protein of the composition of the present disclosure, the numbers of cells secreting MUC5AC were significantly greater than that of the normal control, and at some concentrations, greater than the case treated with Cyclosporine A (0.1 µM, i.e., 120.261 ng/ml).

### [Example 7]

### Preparation of Eye Drop Including HAPLN1 Protein of Present Disclosure

Dye drop preparations were respectively prepared according to a common eye drop preparation method using the ingredients shown in Table 1 below except for the HAPLN1 protein, as a main ingredient of the present disclosure, and evaluated by various tests, i.e., structural analysis (UPLC), functional analysis (Linking ability assay), accelerated harsh conditions, and stability test to evaluate whether the functions of the composition of the present disclosure were properly obtained. According to the results, a pharmaceutical composition including the HAPLN1 protein according to of the present disclosure was prepared into three types of eye drop preparations shown in Table 2 below. In this regard, sodium carboxymethylcellulose (CMC-Na) was used as a viscomodifier, Tris HCl osmotic pressure regulator was used as a buffer, and NaCl was used as an isotonic agent to establish the preparations.

The concentrations of the HAPLN1 protein in the composition of the present disclosure were identical to those of Preparation Examples 1, 2, and 3, i.e., three types of low concentration (0.5 µg/ml), medium concentration (5 µg/ml), and high concentration (50 µg/ml), in all transparent solutions.

**[Table 1]**

| | |
|---|---|
| Viscomodifier | Polyvinyl alcohol (PVA) |
| Viscomodifier | poloxamer |
| Viscomodifier | PVP K-30 |
| Viscomodifier | sodium carboxymethylcellulose |
| Viscomodifier | hydroxypropyl methylcellulose |
| Stabilizer | sodium edetate |
| Stabilizer | lysin |
| stabilizer | arginine |
| Stabilizer | HP-β-CD |
| Stabilizer | sorbitol |
| Buffer | aminocaproic acid |
| Preservative | benzalkonim chloride |
| Isotonic agent | sodium chloride |
| Isotonic agent | mannitol |
| pH regulator | 0.01 M NaOH |
| pH regulator | 0.01 M HCl |

**[Table 2]**

| Additive | Purpose | Preparation Example 1 | Preparation Example 2 | Preparation Example 3 | Unit |
|---|---|---|---|---|---|
| HAPLN1 protein (pH 8.0) | active ingredient | 0.5 | 5.0 | 50 | µg/ml |
| Osmotic pressure | 240-290 | 267 | 267 | 279 | mOsM/kg |
| CMC-Na | viscomodifier | 10 (w/v%) | | | mg/ml |
| Tris HCl | buffer | 5 | | | mM |
| NaCl | osmotic pressure regulator | 125 | | | mM |
| Tween80 | stabilizer (surfactant) | 0.025 | | | % |
| pH | ∼7.4 | 7.72 | | | |

| | | | | | |
|---|---|---|---|---|---|
| *CMC-Na (Sodium carboxymethyl cellulose): Ingredient holding moisture to thicken the tear layer and coat the surface of the eyeball. * In the composition, the balance of sterilized distilled water was used. | | | | | |

### [Example 8]

### Evaluation 1 of Efficacy of HAPLN1 protein-containing Eye Drop Preparation of Present Disclosure in In Vivo Dry Eye Model

Corneal fluorescein score assay and tear volume assay were respectively performed on eyeballs of mice treated with the compositions of Preparation Examples 1, 2, and 3 prepared in Example 7 above by using a low-humidity + scopolamine-induced dry eye disease (DED) model.

### 1. Low-humidity + Scopolamine-induced Dry Eye Disease Model

### Mouse, C57BL/6, female, 8-week-old 96 female mice

Number and age of animal at the time of start of dry eye disease test: Mouse, C57BL/6, female, 10-week-old 58 female mice
When obtaining experimental animals, inspection and quarantines were carried out by referring the microbiological monitoring report of a test system provided by the supplier and observing the appearance of the experimental animals. Animals with no apparent abnormalities were brought into a vivarium and acclimated for 2 weeks in an animal room where tests were conducted. During the quarantine and acclimation period of 2 weeks, health condition and suitability for the experiment were evaluated, and then healthy animals were selected. By using only the animals with no abnormalities during the quarantine and acclimation period, dry eye disease was induced and the degree of corneal damage was evaluated by corneal fluorescein staining, and the test groups were classified by a Z-array method according to clinical scores. A dry eye disease test group consisted of eyeballs with a corneal fluorescein stain score of 7 to 15, and a sham group (normal) consisted of eyeballs not having a corneal fluorescein stains core of 5 or more. For reference, 5 µL of 0.05 % Restasis was administered as an immunosuppressor that suppresses production of T cells, as a control substance, and the concentration thereof was 500 µg/mL.

**[Table 3]**

| Group | Substance administered | Induction of dry eye disease | Dose and frequency | N |
|---|---|---|---|---|
| G1. Non-induced group | Vehicle | X | 5 µL, b.i.d | 8 |
| G2. excipient | Vehicle | O | 5 µL, b.i.d | 8 |
| G3. control substance | Restasis^{®} | O | 5 µL, b.i.d | 8 |
| G4. rhHAPLN1-low | rhHAPLN1 0.5 µg/ml | O | 5 µL, b.i.d | 8 |
| G5. rhHAPLN1-mid | rhHAPLN1 5 µg/ml | O | 5 µL, b.i.d | 8 |
| G6. rhHAPLN1-high | rhHAPLN1 50 µg/ml | O | 5 µL, b.i.d | 8 |

### 2. Induction of Dry Eye Disease

While the test animals were raised in a desiccating stress chamber with a relative humidity of 30 % or less, scopolamine hydrobromide (S0929, Sigma-aldrich) prepared in a concentration of 2.5 mg/mL in saline (0.9% normal saline, JW Pharmaceuticals Corp.) was subcutaneously injected thereinto four times a day (QID: 09:30 AM, 12:00 PM, 15:00 PM, and 17:30 PM) for 10 days at a dose of 10 mL/kg to induce dry eye disease. The subcutaneous injection was performed at the back while moving positions to minimize damage caused by repeated administration. After the administration of the test substance was started, scopolamine was subcutaneously injected around the neck once a day (QD: 09:30 AM) while raising the mice under relative humidity conditions of 30 % or less to maintain the dry eye disease.

### 3. Administration Method and Frequency of Administration

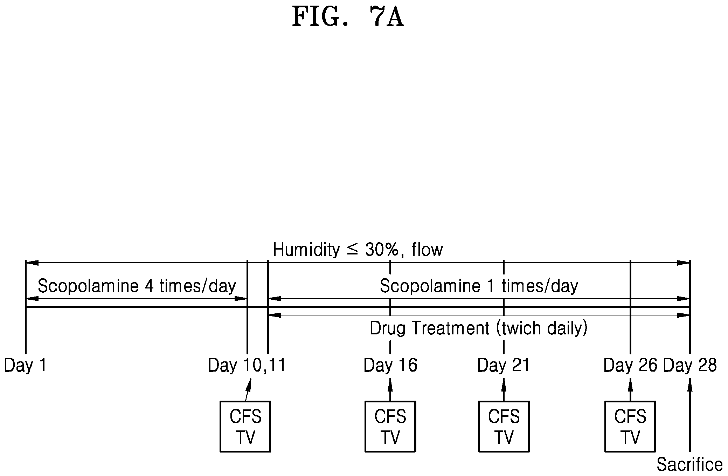

From the next day of group classification, one drop (5 µl) of each of the test substance and the control substance was instilled into both eyes twice a day (BID: 09:30 AM and 17:30 PM) using a micropipette (FIG. 7A).

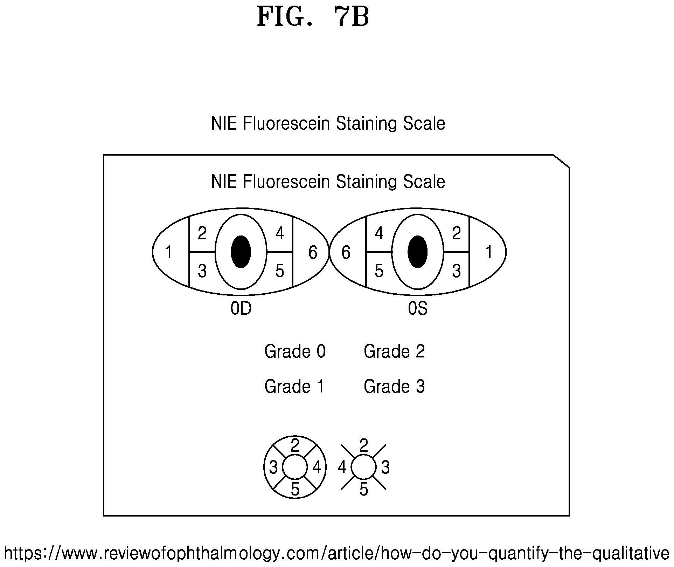

### 4. Corneal Fluorescein Score Assay (FIG. 7B)

(1) One drop of a mydriatic agent (Tropaeolin) was administered to induce dilation of the pupil, and the remaining mydriatic agent was removed by placing a cotton swab on the tips of canthi.
(2) 10 µl of 0.2% fluorescein sodium salt was administered to each of both eyes for fluorescent staining damaged regions.
(3) After lightly washing the eyeballs with physiological saline, cornea was imaged under blue light. According to guidelines of the National Institute of Eye (NIE), the area of the cornea was divided into 5 sections and each section was scored from 0 to 3 points, and then the values were summed to calculate a fluorescein score.

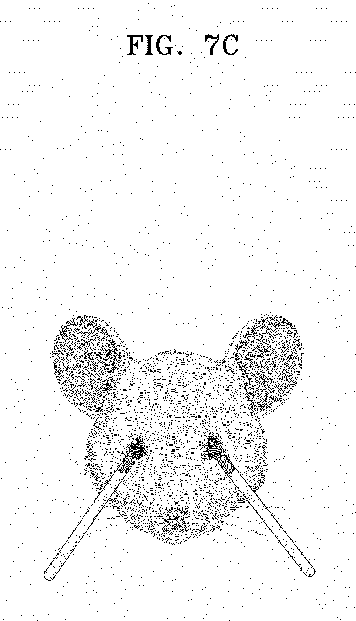

### 5. Tear Volume Assay (FIG. 7C)

(1) General anesthesia of the mice was induced (10 mg/kg xylazine and 100 mg/kg ketamine, intraperitoneal injection).
(2) After 3 hours from administration of scopolamine hydrobromide in the morning (at 9:30 AM), measurement was conducted.
(3) The volume of tear was measured by using a Schirmer's test strip cut to 1/2 thickness. The Schirmer test strip was applied to be in contact with the inner side of the lower eyelid and maintained such that tear was absorbed for 5 minutes. Then, one drop (5 µl) of physiological saline was applied to the eyeballs.
(4) A length of a wet portion of the strip was measured by a ruler.

### 6. Details of Experiment

### A. Measurement of Weight

Weight of all animals was measured at the time of group classification (Day 0) and at every evaluation (on the 6^{th} day, 11^{th} day, and 16^{th} day from the start of administration).

### B. Observation of General Symptoms

Clinical observation (cage-side observation) including observation of conditions of eyeballs was performed at least once a day on all animals from the time of administration of the test substance to the end of the experiment.

### C. Measurement of Tear Volume (Schirmer's test) and Clinical Evaluation of Corneal Damage (Corneal Fluorescein Score)

After dry eye disease was induced by applying desiccating stress and administering scopolamine hydrobromide, evaluation was performed by inducing general anesthesia by intraperitoneally injecting 10 mg/kg xylazine (Rompun, Bayer) and 100 mg/kg ketamine (Yuhan Corporation) at the time of group classification (Day 0) and on the 6^{th} day, 11^{th} day, and 16^{th} day from the start of administration of the test substance. If anesthesia is not sufficiently induced, additional anesthesia was induced by intramuscularly injecting 1/3 of the dose used in the initial induction of anesthesia into the thigh of the mice (In consideration of characteristics of injectectable anesthetics, the animals may die during the anesthesia-inducing process). After 3 hours from administration of scopolamine hydrobromide in the morning (at 9:30 AM), evaluation was conducted. The tear volume was measured using a Schirmer's test strip (Bio Color Tear Test, Bio Optics) cut to 1/2 thickness. The Schirmer test strip was applied to be in contact with the inner side of the lower eyelid and maintained for 5 minutes such that tear was absorbed. After the evaluation was completed, one drop (5 µL) of physiological saline was applied to the eyeballs. A length of a wet portion (dyed portion) of the strip was measured by a ruler. Subsequently, one drop of a mydriatic agent (Tropaeolin, Alcon Korea) was applied thereto to induce mydriasis, and the mydriatic agent remaining on the eyeballs was removed by placing a cotton swab on the tips of canthi. Subsequently, 10 µL of 0.2 % fluorescein sodium salt (Fluorescite, Alcon Korea) was administered to each of both eyes for fluorescent staining of damaged regions. Then, after lightly washing the eyeballs with physiological saline, cornea was imaged under blue light (Micron-IV, Phoenix). According to guidelines of the National Institute of Eye (NIE), the area of the cornea was divided into 5 sections and each section was scored from 0 to 3 points, and then the values were summed to calculate a fluorescein score. The anesthesia, measurement of tear volume, and CFS evaluation were completed within 15 minutes. Upon completion of the evaluation, one drop of an antibiotic eye drop (Toberan Eye Drop, Daewoo Pharmaceuticals Co., Ltd.) was administered to the eyeballs of the mice.

### D. Eye Enucleation

On the 18^{th} day after the administration was started, lacrimal gland, cornea, conjunctiva, and meibomian gland (meibomian gland-containing lower eyelid) of both eyes were sampled and provided to a client in a frozen state). However, upon a request of a client, enucleation of both eyes was performed to include upper and lower eyelids, immobilized with Davidson's sol. and formalin, and then prepared into paraffin tissue slides instead of using the above-described method.

### E. Statistical Analysis of Data

All test results obtained in the experiment were expressed as average ± standard deviation and analyzed using GraphPad Prism 6.0 (GraphPad Software, CA). Normality test of all data was performed by the Shapiro-wilk normality test. In the case of following normal distribution, one-way analysis of variance (ANOVA) was carried out. Once significance was observed, the Tukey's multiple comparison test was conducted to detect a test group with a significant difference from that of the control (excipient). In the case of not following normal distribution, the Kruskal Wallis test was performed and the Dunn's multiple comparisons test was performed for a post-hoc test (significance level: both sides 5 % and 1 %).

### 7. Results of Experiment

### (1) Evaluation of Degree of Corneal Damage in Mouse Dry Eye Model by Corneal Fluorescein Score (CFS)

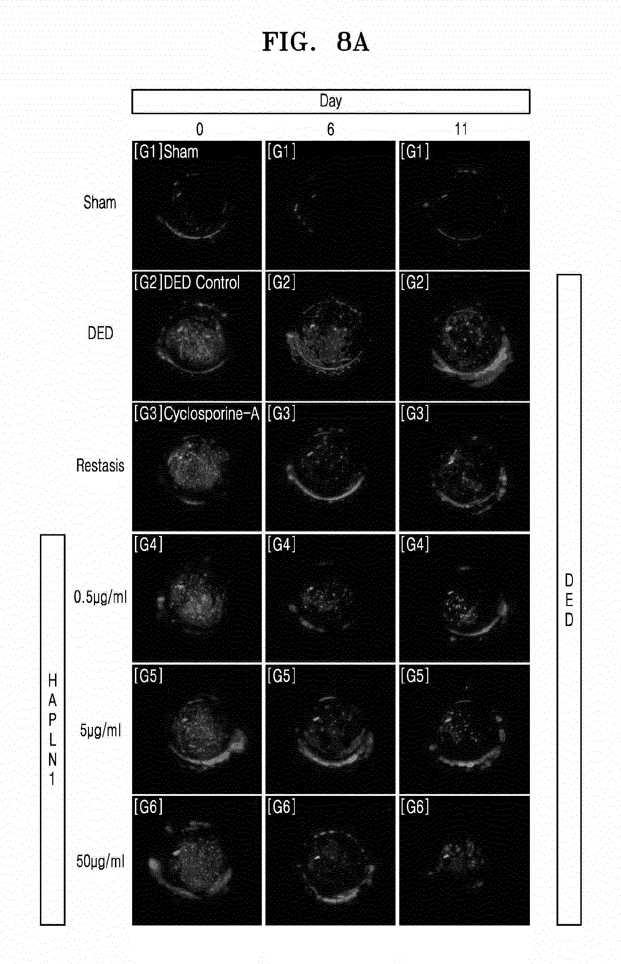

Imaging results of a corneal fluorescein score assay are shown in FIG. 8A. Here, the Sham refers to a normal control model in which the dry eye disease was not induced, the DED control refers to an untreated control model in which the dry eye disease was induced and the cells were treated with a vehicle not including a drug, and the Cyclosporine A refers to an example treated a conventional therapeutic agent. The rhHAPLN1 (0.5 µg/ml) refers to an example treated with the composition of the present disclosure according to Preparation Example 1, the rhHAPLN1 (5 µg/ml) refers to an example treated with the composition of the present disclosure according to Preparation Example 2, and the rhHAPLN1 (50 µg/ml) refers to an example treated with the composition of the present disclosure according to Preparation Example 3.

In this case, one drop (5 µl) of each of the test substance and the control substance was administered to both eyes twice a day (BID: 09:30 AM and 17:30 PM) by using a micropipette from the next day of group classification as described above in 3.

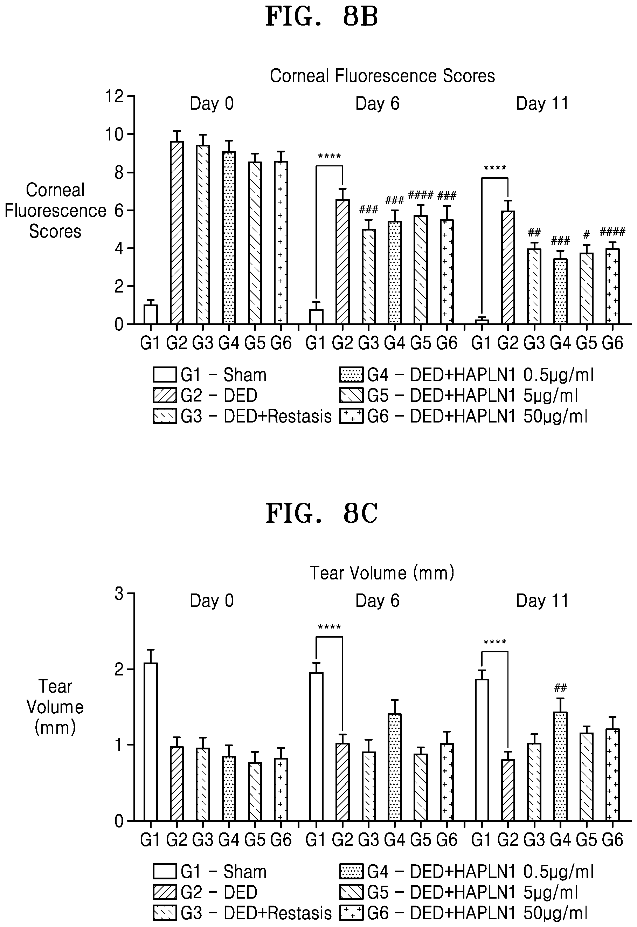

The results of the corneal fluorescein score assay were quantified and shown in FIG. 8B as a graph.

According to the administration results, the CFS level increased in the dry eye disease model consistently decreased by treatment with the HAPLN1 protein-containing composition of the present disclosure regardless of the concentration. Particularly, after 11 days, the HAPLN1 protein-composition of the present disclosure reduced the CFS level more than Cyclosporine A (main ingredient of Restasis as a control drug).

### (2) Evaluation of Tear Volume in Mouse Dry Eye Model

Results of tear volume assay were quantified and shown in FIG. 8C.

According to the results of administration, the tear volume reduced in the dry eye disease model was consistently increased by treatment with the HAPLN1 protein-containing composition of the present disclosure regardless of the concentration more than by treatment with Cyclosporine A (main ingredient of Restasis as a control drug). Particularly, after 11 days, the HAPLN1 protein-containing composition of the present disclosure increased the tear volume far more than Cyclosporine A.

### (3) Evaluation of Detachment of Corneal Epithelial Cell

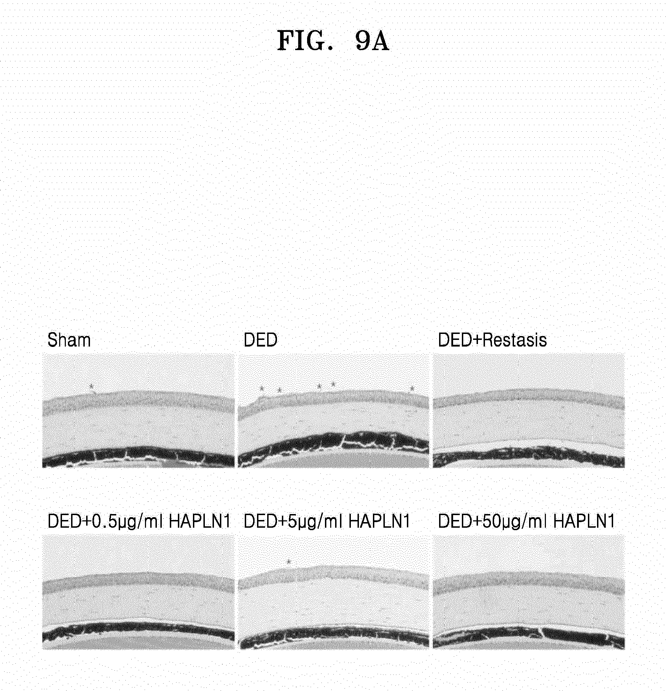

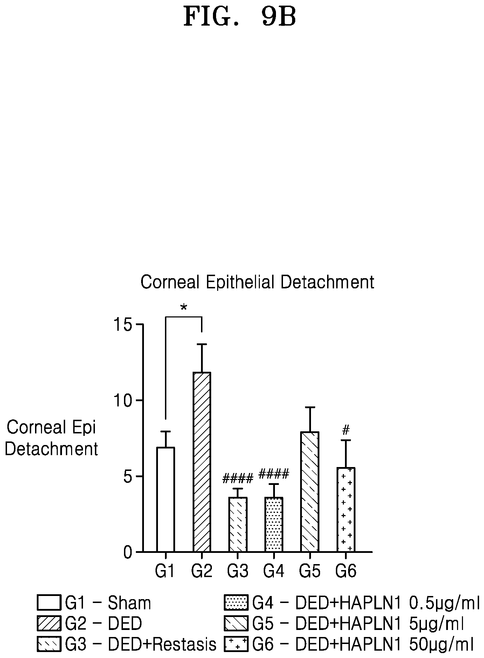

On the 2^{nd} day after 16 days of drug treatment, eyes were enucleated and paraffin blocked, and tissue was stained by hematoxylin and eosin (H & E) staining on a prepared slide (FIG. 9A). The number of detached corneal epithelial cells was measured and quantified (FIG. 9B).

As a result, the number of damaged corneal epithelial cells increased in the dry eye disease model. However, it was confirmed that the number of damaged cells decreased by treatment with the HAPLN1 protein-containing composition of the present disclosure regardless of concentration.

### (4) Evaluation of Increase or Decrease of Goblet Cell

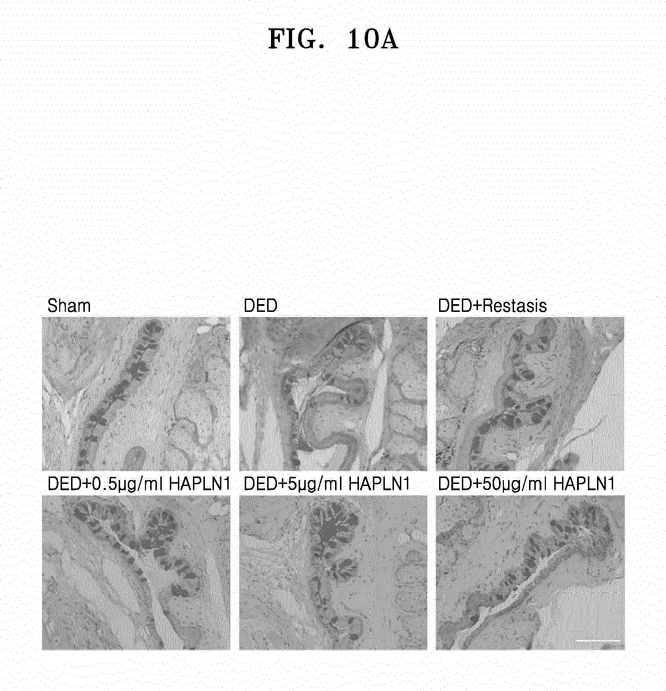

On the 16^{th} days from drug treatment, eyes were enucleated and paraffin blocked, and tissue was stained by PAS staining on a prepared slide (FIG. 10A). PAS staining specifically stains goblet cells. Because there are many mucopolysaccharides and mucoproteins in the goblet cells, α=glycol group thereof is oxidized by a periodic acid to form an aldehyde, which reacts with a Schiff reagent to be stained in red-purple.

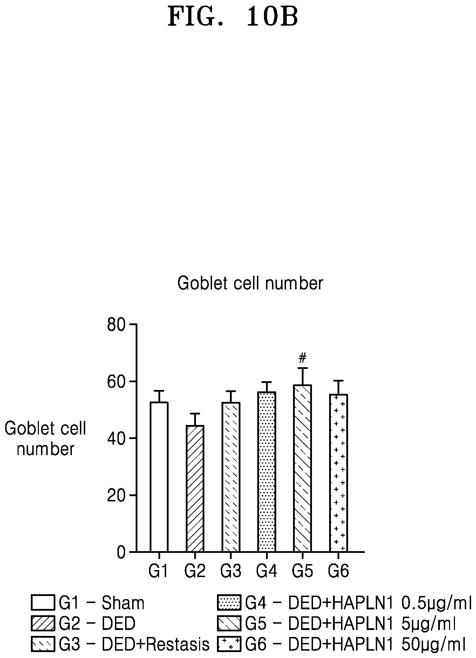

In this regard, the number of goblet cells present in the conjunctiva was measured and quantified (FIG. 10B). As a result, in the dry eye disease model, the number of damaged goblet cells decreased. However, it was confirmed that the number of goblet cells significantly increased by treatment with the HAPLN1 protein-containing composition of the present disclosure regardless of the concentration compared to the dry eye model, and the number of goblet cells was equivalent to that of the normal Sham control.

### (5) Evaluation 1 of Anti-inflammatory Activity in Lacrimal Gland

Once dry eye disease is induced, inflammatory response is induced in eyeballs and lacrimal glands, causing an increase in IL-1β that is a representative cytokine of inflammatory response and occurrence of invasion of immune cells.

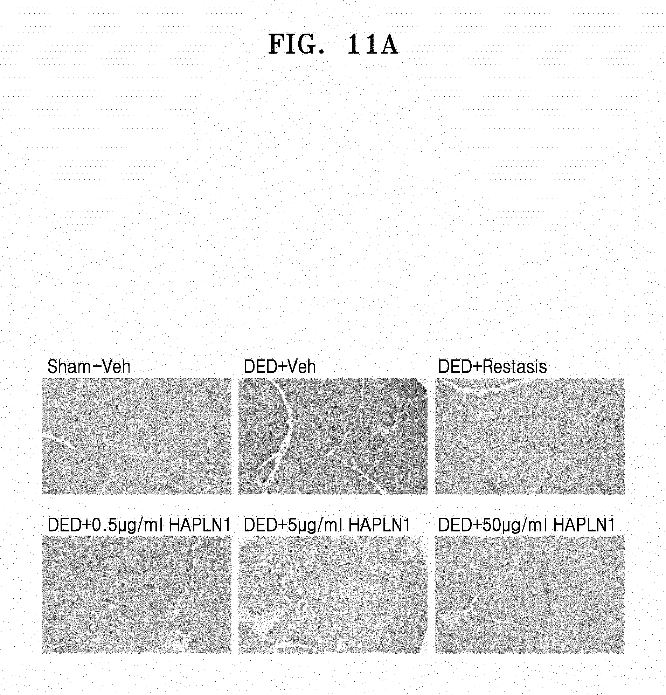

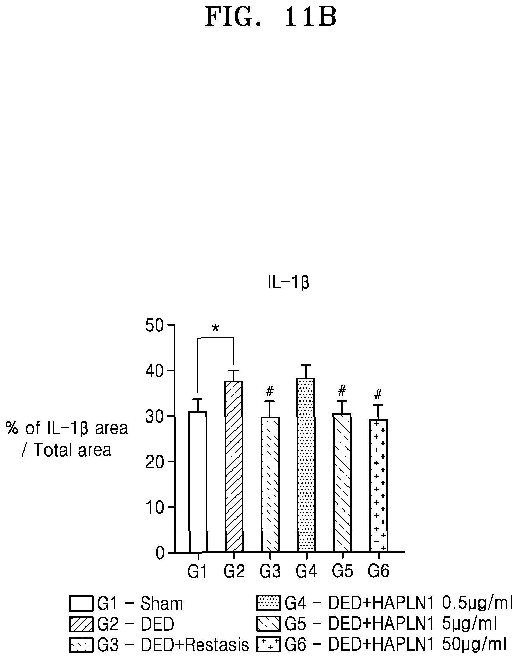

In this example, expression of IL-1β in lacrimal glands was identified by immunohistochemistry in order to identify an in vivo anti-inflammatory effect of the HAPLN1 protein-containing composition of the present disclosure. To this end, on the 2^{nd} day after 16 days of drug treatment, eyes were enucleated and paraffin blocked. IHC was performed by using a prepared slide, and expression (brown) of IL-1β was identified by using an Anti-IL1β antibody (FIG. 11A). FIG. 11B is a graph illustrating quantified expression levels of IL-1β obtained in FIG. 11A.

As a result, in the dry eye disease model, expression of IL-1β increased in the lacrimal gland. However, the expression of IL-1β decreased to a level similar to that of Sham control corresponding to normal cells in the case where the cells were treated with the HAPLN1 protein-containing composition of the present disclosure at a concentration of 5 µg/ml or more of HAPLN1. This proves anti-inflammatory effect of the composition of the present disclosure.

### (6) Evaluation 2 of Anti-inflammatory Activity in Lacrimal Gland

Once dry eye disease is induced, inflammatory response is induced in eyeballs and lacrimal glands, causing an increase in expression of CD45, which is an inflammatory marker in immune cells, on the surfaces of cells indicating invasion of the immune cells.

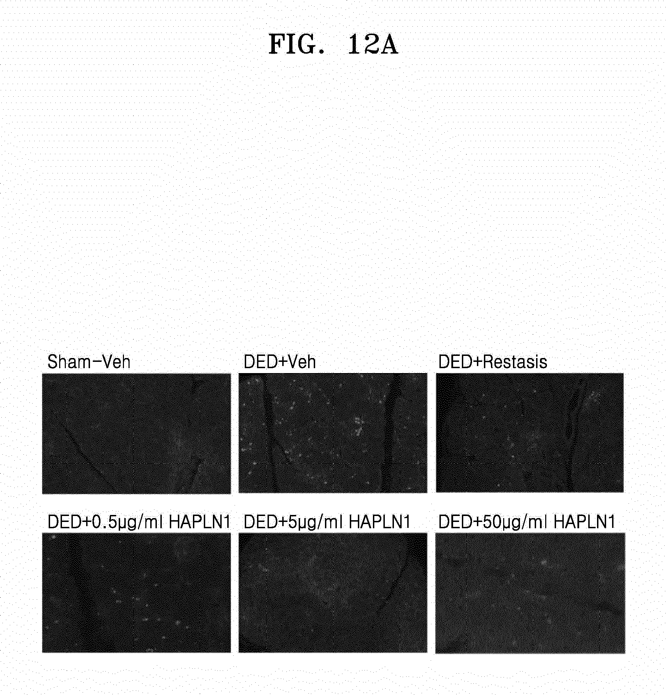

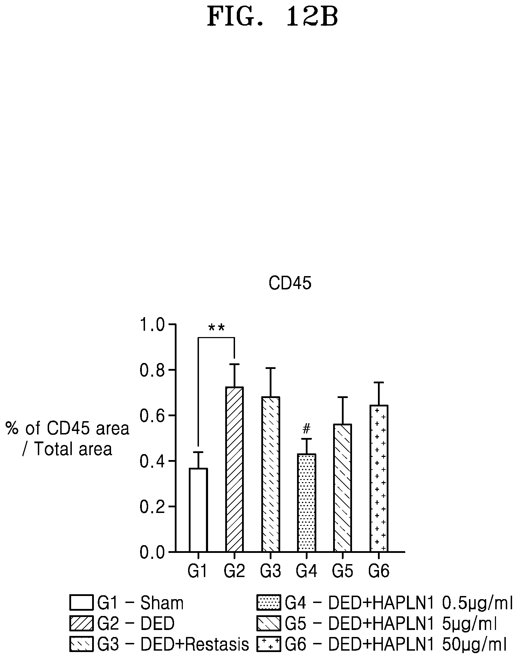

In this example, expression of CD45 was identified in lacrimal glands in order to identify in vivo anti-inflammatory effect of the HAPLN1 protein-containing composition of the present disclosure. That is, on the 2^{nd} day after 16 days of drug treatment, eyes were enucleated and paraffin blocked. IHC-IF was performed on a section slide, and expression (green) of CD45 was identified by using an Anti-CD45 antibody. FIG. 12B is a graph illustrating quantified expression levels of FIG. 12A.

As a result, in the dry eye disease model, expression levels of CD45 increased in the lacrimal glands. However, the expression of CD45 decreased by treatment with the HAPLN1 protein-containing composition of the present disclosure. Particularly, the HAPLN1 protein-containing composition of the present disclosure reduced the expression level of CD45 more than Cyclosporine A (main ingredient of Restasis as a control drug). This proves anti-inflammatory effect of the composition of the present disclosure.

### (7) Evaluation 3 of Anti-inflammatory Activity in Lacrimal Gland

Once dry eye disease is induced, inflammatory response is induced in eyeballs and lacrimal glands, and an increase in expression of CD11b as an inflammatory marker in immune cell indicates invasion of the immune cells.

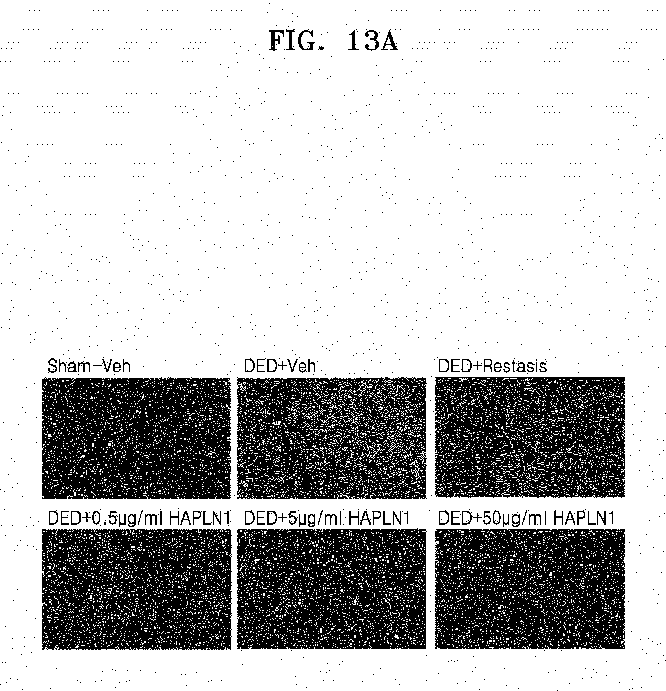

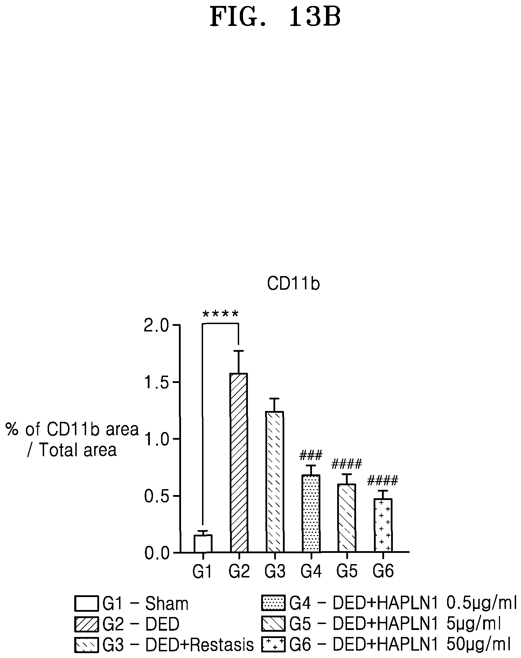

In this example, expression of CD11b was identified in lacrimal glands in order to identify in vivo anti-inflammatory effect of the HAPLN1 protein-containing composition of the present disclosure. That is, on the 2^{nd} day after 16 days of drug treatment, eyes were enucleated and paraffin blocked. IHC-IF was performed on a section slide, and expression (green) of CD11b was identified by using an Anti-CD11b antibody. FIG. 13B is a graph illustrating quantified expression levels of FIG. 13A (FIG. 13B).

As a result, in the dry eye disease model, expression levels of CD11b increased in the lacrimal glands. However, the expression of CD11b decreased by treatment with the HAPLN1 protein-containing composition of the present disclosure. Particularly, the HAPLN1 protein-containing composition of the present disclosure reduced the expression level of CD11b more than Cyclosporine A (main ingredient of Restasis as a control drug). This proves anti-inflammatory effect of the composition of the present disclosure.

### [Example 9]

### Evaluation 2 of Efficacy of HAPLN1 protein-containing Eye Drop Preparation of Present Disclosure in In Vivo Dry Eye Model

Corneal fluorescein score assay and tear volume assay were respectively performed on eyeballs of rabbits treated with the compositions of Preparation Examples 1, 2, and 3 prepared in Example 7 above by using a 0.1 % benzalkonium chloride (BAC)-induced dry eye disease (DED) model.

### 1. Establishment of Dry Eye Disease Model for Rabbit

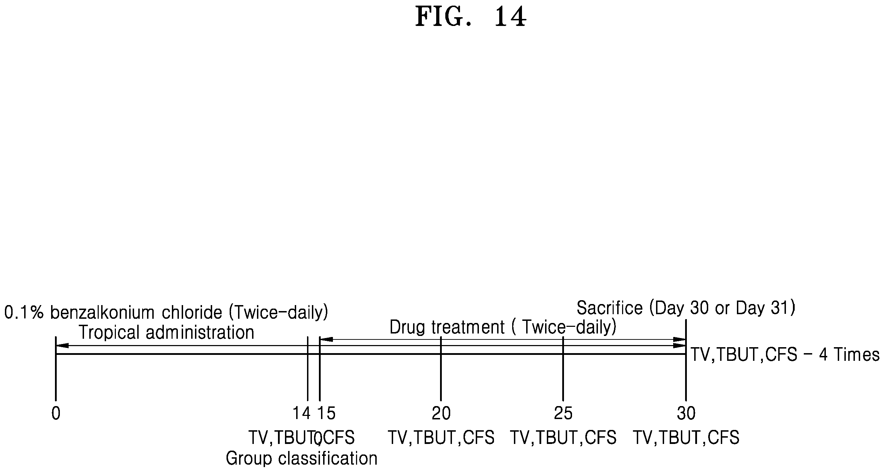

A dry eye disease-inducing model was established for rabbits according to the following order of an experiment (FIG. 14) and efficacy of the composition of the present disclosure was verified.

### (1) Induction of Dry Eye

One drop (0.05 ml) of 0.1% benzalkonium chloride (BAC) was administered to both eyes of rabbits twice a day (9:00 and 17:00) for 14 days, and then induction of dry eye was identified by examination of eyes, and the rabbits were classified into groups.

### (2) Instillation of Test Substance

The test substance was dropped into the groups of the rabbit model twice a day (8:30 and 16:30) (Day 15 to 30 (total of 16 days)).

**[Table 4]**

| | | Substance administered | Induction of dry eye disease | Dose and frequency | N |
|---|---|---|---|---|---|
| G1 | Non-induced group | Vehicle | X | 5 µl, b.i.d | 7 |
| G2 | Induced group | Vehicle | O | 5 µl, b.i.d | 7 |
| G3 | Control drug 1 | Restasis | O | 5 µl, b.i.d | 7 |
| G4 | Test drug 1 | 0.5 µg/ml HAPLN1 | O | 5 µl, b.i.d | 7 |
| G5 | Test drug 2 | 5 µg/ml HAPLN1 | O | 5 µl, b.i.d | 7 |
| G6 | Test drug 3 | 50 µg/ml HAPLN1 | O | 5 µl, b.i.d | 7 |

### Experimental animals: 42 New Zealand white rabbits (Male, individually bred)

### Group classification:

G1. No DED with vehicle (Dry eye-non-induced group)
G2. DED with vehicle (Dry eye-induced group)
G3. DED with Restasis (Cyclosporine)
G4. DED with test substance (0.5 µg/ml HAPLN1 Dose-1) G5. DED with test substance (5 µg/ml HAPLN1 Dose-2)
G6. DED with test substance (50 µg/ml HAPLN1 Dose-3)
*All groups were euthanized on the 2^{nd} day after 16 days from instillation
*All groups were administered with the eye drop twice a day
A total of 42 rabbits were used (7 per each group)

### (3) Eye Examination

Anesthesia was induced by intramuscular injection of Xylazine and Ketamine, and then one drop of Alcaine Eye Drop was administered and tear and the eye drop remaining therein were removed by using Weck-cel cellulose spear. After putting STT paper on the lower eyelid, a length of a wet region for 5 minutes was measured (STT-2). In addition, stability of the tear film was tested by measuring tear film break up time (TFBUT, measurement of time taken for the tear film to break down). Furthermore, conditions of the cornea and fluorescent staining were identified by a slit test.

### (4) Sampling and Analysis

Eyes of the rabbits including conjunctiva were enucleated, fixed with a specific fixing solution to be embedded into paraffin, and microsectioned, followed by staining and analysis according to the purpose. In this regard, the eyeballs were stained by hematoxylin & eosin (H & E) staining and detachment of corneal epithelial tissue was analyzed. Afte PAS staining, distribution of goblet cells in the conjunctiva was measured. In addition, after conjunctiva epithelium indentation (impression cytology), PAS staining was performed and distribution of goblet cells was measured.

### 2. Test Result

### (1) Evaluation of Decree of Corneal Damage with Corneal Fluorescein Score (CFS)

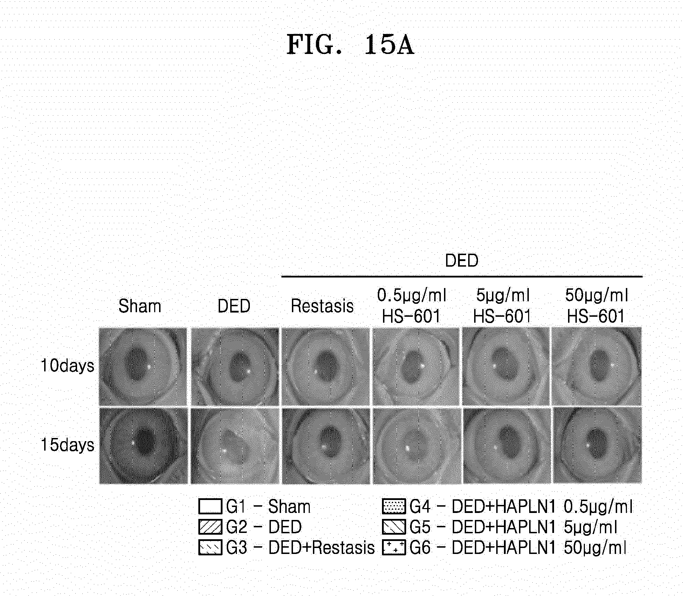

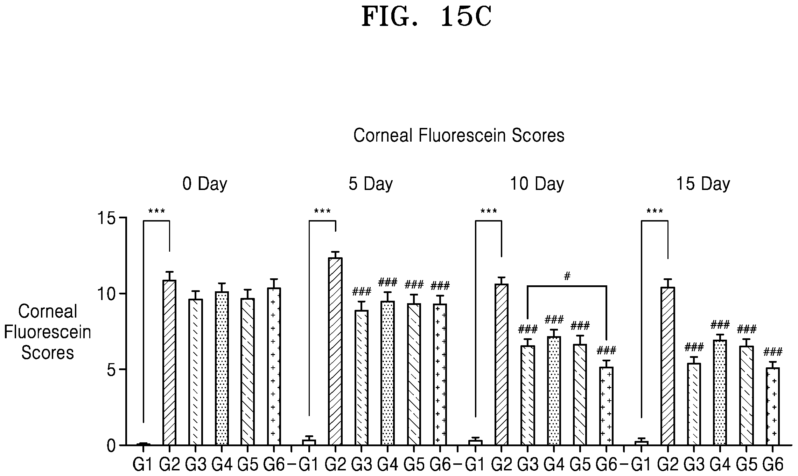

Imaging results of a corneal fluorescein score assay are shown in FIG. 15A and a graph showing quantified results is shown in FIG. 15C. Based on the results of administration, the CFS level that increased in the dry eye disease model consistently decreased by treatment with the HAPLN1 protein-containing composition of the present disclosure regardless of the concentration. Particularly, after 10 days, the CFS level was reduced by the HAPLN1 protein-containing composition of the present disclosure more than by Restasis.

### (2) Evaluation of Efficacy of Composition of Present Disclosure on Improvement of Stability of Tear Film

In this example, a method of measuring time until black spots appeared at fluorescent stained regions after applying one drop of sodium fluorescein to eyeballs. The black spots may appear within several seconds after blinking and occurs very quickly under the conditions of dry eye disease. That is, stability of the tear film was measured by measuring the tear film break up time (TFBUT, measurement of time taken for the tear film to break down).

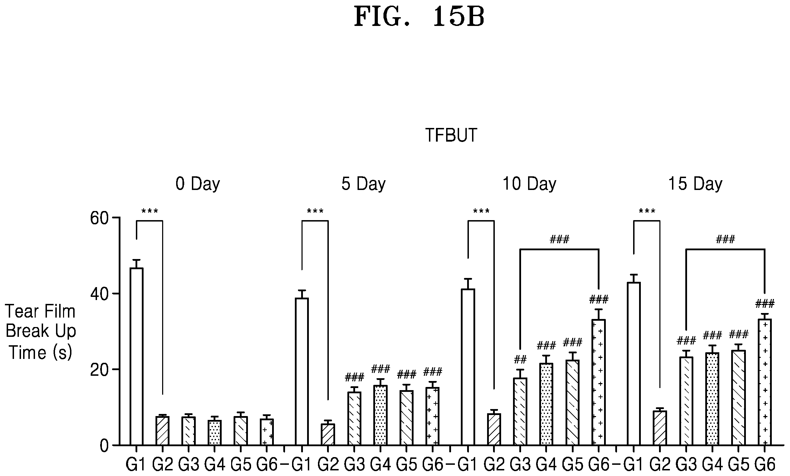

TFBUT of each sample was measured and the results are shown as a graph (FIG. 15B). Here, the Sham refers to a normal control model in which the dry eye disease was not induced, the DED control refers to an untreated control model in which the dry eye disease was induced and the cells were treated with a vehicle not including a drug, and the Cyclosporine A refers to an example treated a conventional therapeutic agent, and Restasis (wherein active ingredient is Cyclosporine A) refers to an example treated a conventional therapeutic agent. The HAPLN1 (0.5 µg/ml) refers to an example treated with the composition of the present disclosure according to Preparation Example 1, HAPLN1 (5 µg/ml) refers to an example treated with the composition of the present disclosure according to Preparation Example 2, and HAPLN1 (50 µg/ml) refers to an example treated with the composition of the present disclosure according to Preparation Example 3. In FIG. 15A, HS-601 indicates the title of the experiment of the HAPLN1 protein-containing composition of the present disclosure.

As shown in FIG. 15B, according to results of the administration, although the tear film break up time of the dry eye disease model was very short less than 10 seconds, the TFBUT continuously increased in the case of administration of the composition of the present disclosure, and particularly, after 10 days, the TFBUT was increased by the HAPLN1 protein-containing composition of the present disclosure, regardless of the concentration of HAPLN1, more than by Restasis. Particularly, it was confirmed that the TFBUT was increased to 30 seconds or more at a high concentration (50 µg/ml) of HAPLN1 protein.

### (3) Evaluation of Tear Volume in Rabbit Dry Eye Model

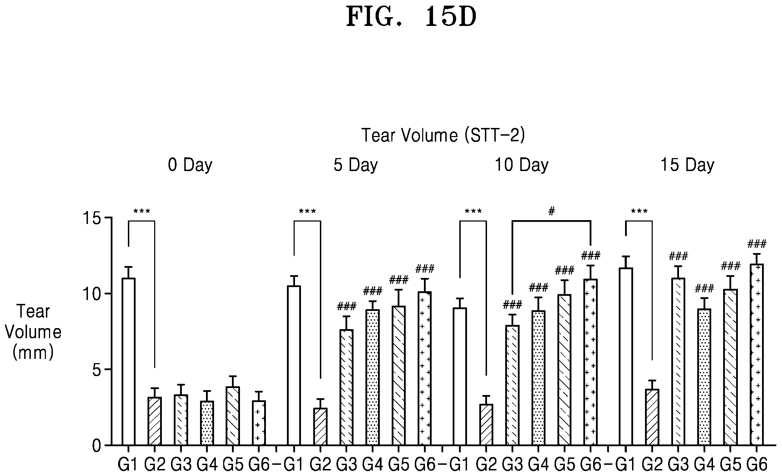

Results of tear volume assay were quantified and shown in FIG. 15D.

According to the results of administration, the tear volume reduced in the dry eye disease model was consistently increased by treatment with the HAPLN1 protein-containing composition of the present disclosure in a concentration-dependent manner from the 5^{th} day more than by treatment with Restasis as a control drug. Particularly, after 10 days, the HAPLN1 protein-containing composition of the present disclosure increased the tear volume far more than Restasis.

### (4) Evaluation of Detached State of Corneal Epithelial Cell

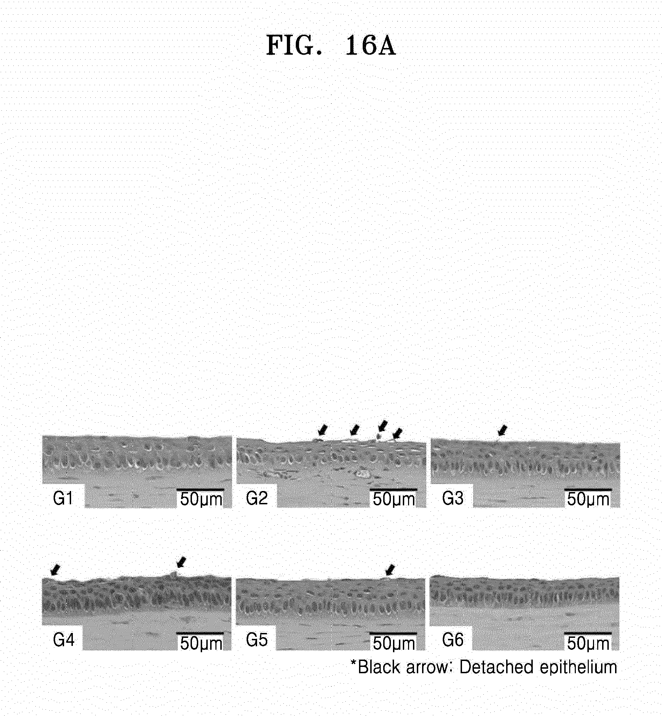

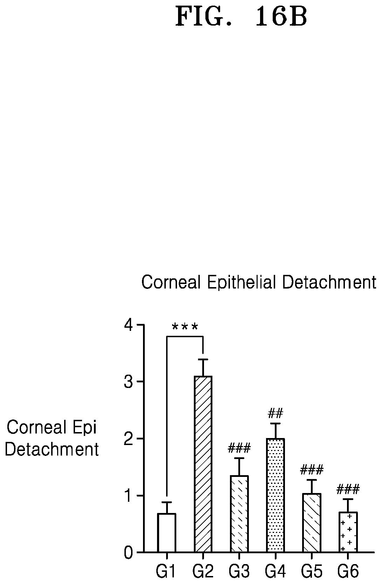

After 15 days of eye examination, on the 1^{st} day after 16 days of drug treatment, eyes were enucleated, subjected to histopathological analysis, and paraffin blocked. Tissue was stained in a section slide by H & E staining (FIG. 16A). The number of corneal epithelial cells detached in a range of 200 µm² was measured and quantified (FIG. 16B).

As a result, the number of detached, i.e., damaged, corneal epithelial cells increased in the dry eye disease model. However, it was confirmed that the number of detached cells decreased in the case of treatment with the HAPLN1 protein-containing composition of the present disclosure in a concentration dependent manner. Therefore, the degree of detachment was the lowest in the high concentration (50 µg/ml) of the HAPLN1 protein of the composition of the present disclosure.

### (5) Evaluation of Increase or Decrease of Goblet Cell in Rabbit Dry Eye Model

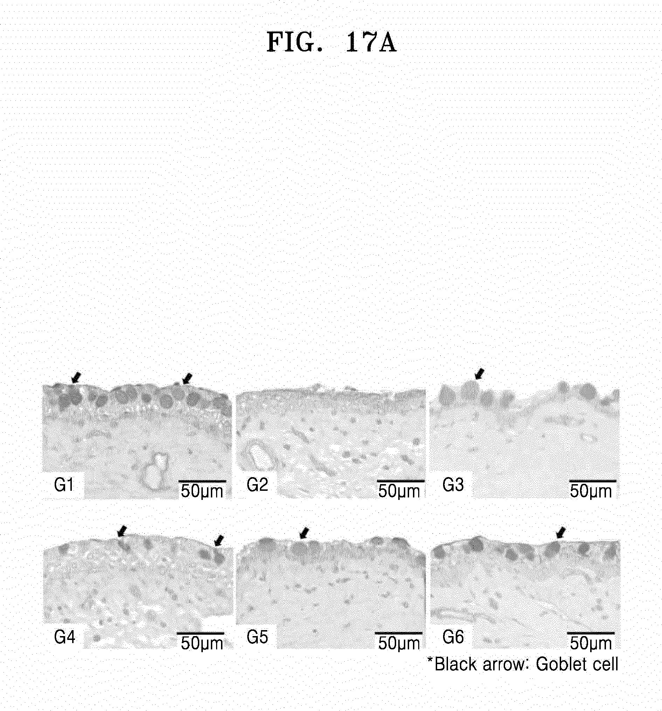

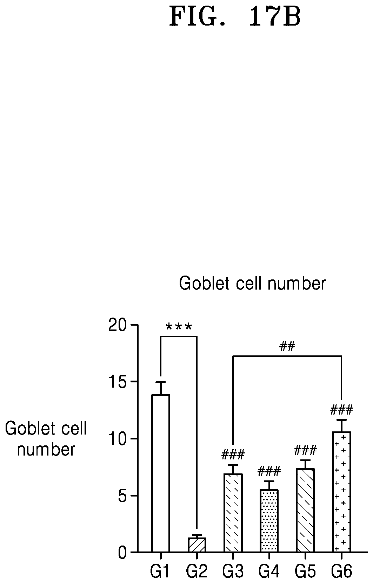

After 15 days of eye examination, on the 1^{st} day after 16 days of drug treatment, eyes were enucleated, subjected to histopathological analysis, and paraffin blocked. Tissue was stained in a section slide by PAS staining (FIG. 17A). The number of goblet cells in a range of 200 µm² was measured and quantified (FIG. 17b).

As a result, the number of damaged goblet cells was extremely decreased in the dry eye disease model. However, it was confirmed that the number of goblet cells was significantly increased by treatment with the HAPLN1 protein-containing composition of the present disclosure in proportion to the concentration compared to the dry eye disease model. However, it was confirmed that the number of goblet cells was far more increased in the case of the high concentration (50 µg/ml) of the HAPLN1 protein of the composition of the present disclosure compared to Restasis as a control drug.

### Industrial Applicability

According to the pharmaceutical composition and treatment method of the present disclosure as described above, the dry eye disease may be effectively and stably prevented and treated with reduced side effects compared to conventional products.

### Sequence List Text

SEQ ID NO: 1:

MKSLLLLVLISICWADHLSDNYTLDHDRAIHIQAENGPHLLVEAEQAKVFSHRGGNVTL
PCKFYRDPTAFGSGIHKIRIKWTKLTSDYLKEVDVFVSMGYHKKTYGGYQGRVFLKGG
SDSDASLVITDLTLEDYGRYKCEVIEGLEDDTVVVALDLQGVVFPYFPRLGRYNLNFHE
AQQACLDQDAVIASFDQLYDAWRGGLDWCNAGWLSDGSVQYPITKPREPCGGQNTV
PGVRNYGFWDKDKSRYDVFCFTSNFNGRFYYLIHPTKLTYDEAVQACLNDGAQIAKV
GQIFAAWKILGYDRCDAGWLADGSVRYPISRPRRRCSPTEAAVRFVGFPDKKHKLYG
VYCFRAYN

## Claims

1. A pharmaceutical composition for preventing or treating dry eye disease, comprising hyaluronan and proteoglycan link protein 1 (HAPLN1) or a gene encoding HAPLN1 as an active ingredient.

2. The pharmaceutical composition of claim 1, wherein the protein has a sequence identity of 80 % or more to an amino acid sequence of SEQ ID NO: 1.

3. The pharmaceutical composition of claim 1, wherein a nucleic acid of the gene is included in an expression vector.

4. The pharmaceutical composition of claim 1, wherein the composition induces wound healing of cells in an eyeball.

5. The pharmaceutical composition of claim 1, wherein the composition reduces expression of inflammatory factors of a corneal epithelial cell.

6. The pharmaceutical composition of claim 1, wherein the composition increases an expression level of mucin.

7. The pharmaceutical composition of claim 1, wherein a single topical dose of the composition is 1 ng/ml to 100 µg/ml.

8. The pharmaceutical composition of claim 1, wherein the composition is in a formulation selected from the group consisting of eye drops, artificial tears, ophthalmic ointments, tablets, pills, capsules, troches, inhalants, injections, patches, and suppositories.

9. The pharmaceutical composition of claim 1, wherein the pharmaceutical composition is for parenteral administration.

10. The pharmaceutical composition of claim 1, wherein the pharmaceutical composition further comprises a pharmaceutical additive selected from the group consisting of pharmaceutically acceptable carriers, diluents, binders, disintegrants, lubricants, and combinations thereof.

11. The pharmaceutical composition of claim 1, wherein the composition is included as a major or minor active ingredient in a composition for preventing or suppressing dry eye disease.

12. A kit comprising the composition of any one of claims 1 to 11 and administration instructions.

13. A reagent composition for an experiment related to prevention or suppression of dry eye disease, comprising hyaluronan and proteoglycan link protein 1 (HAPLN1) or a gene encoding HAPLN1, used in preparation of a pharmaceutical composition for preventing or suppressing dry eye disease.

14. A use of hyaluronan and proteoglycan link protein 1 (HAPLN1) or a gene encoding HAPLN1, used in preparation of a pharmaceutical composition for preventing or suppressing dry eye disease.

15. A method of preventing or suppressing dry eye disease, the method comprising administering hyaluronan and proteoglycan link protein 1 (HAPLN1) or a gene encoding HAPLN1, as an active ingredient, to an individual.
